# EUROPÄISCHE PATENTSCHRIFT

(11) **EP 1 496 904 B1**
(45) Veröffentlichungstag und Bekanntmachung des Hinweises auf die Patenterteilung: **15.08.2007**
(21) Anmeldenummer: 02796584.7
(22) Anmeldetag: 09.12.2002
(51) Int. Cl.: A61K 31/495, A61K 31/496, A61P 25/04, A61P 25/06, A61P 25/02, A61P 25/28, A61P 25/14, A61P 25/16, A61P 25/08, A61P 25/18, A61P 25/22, A61P 25/24, A61P 15/12, A61P 25/00, A61P 21/00, A61P 27/02, A61P 1/00

(54) **VERWENDUNG VON ARZNEIMITTELN ENTHALTEND N, N'-DISUBSTITUIERTE PIPERAZIN-VERBINDUNGEN**
USE OF PHARMACEUTICALS CONTAINING N, N'-DISUBSTITUTED PIPERAZINE COMPOUNDS
UTILISATION DE MEDICAMENTS CONTENANT DES COMPOSES DE PIPERAZINE N, N'-DISUBSTITUES

(30) Priorität: 14.12.2001 DE 10161809
(43) Veröffentlichungstag der Anmeldung: 19.01.2005
(73) Patentinhaber: Grünenthal GmbH, 52078 Aachen (DE)
(72) Erfinder: URAGG, Heinz, 52223 Stolberg (DE); MAUL, Corinna, 52066 Aachen (DE); BUSCHMANN, Helmut, E-08950 Esplugues de Llobregat (ES); SUNDERMANN, Bernd, 52066 Aachen (DE); HAURAND, Michael, 52078 Aachen (DE); CHIZH, Boris, Whittlesford, Cambridge CB2 4NW (GB)
(74) Vertreter: Kutzenberger, Helga
(86) Internationale Anmeldenummer: PCT/EP2002/013911
(87) Internationale Veröffentlichungsnummer: WO 2003/051369

(56) Entgegenhaltungen:
- NIKOLOVA M ET AL: "SYNTHESIS AND PHARMACOLOGICAL SCREENING OF A GROUP OF PIPERAZINE DERIVATIVES. ANALGESIV ACTIVITY" FARMACO, SOCIETA CHIMICA ITALIANA, PAVIA, IT, Bd. 48, Nr. 4, 1. April 1993 (1993-04-01), Seiten 459-472, XP000617338 ISSN: 0014-827X
- HASHIMOTO M ET AL: "METABOLISM OF A NEW ANALGESIC AGENT, DL-ERYTHRO-1-PHENYL-2-(0-CHLOROP HENYL)-2-4-(P-METHOXYBENZYL)-1- PIPERAZINYLETHANOL DIHYDROCHLORIDE, IN RATS" DRUG METABOLISM AND DISPOSITION, WILLIAMS AND WILKINS., BALTIMORE, MD, US, Bd. 7, Nr. 6, 1979, Seiten 435-441, XP000973712 ISSN: 0090-9556

## Beschreibung

Die vorliegende Erfindung betrifft die Verwendung von N,N'-disubtituierten Piperazin-Verbindungen zur Herstellung eines Arzeimittels zur Behandlung von Erkrankungen wie in den Ansprüchen defininiert. Die Behandlung von Schmerz hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind. Ebenso ist die Behandlung von Epilepsie in der Medizin von großer Bedeutung.

Das cyclische GABA-Analoge Gabapentin ist ein klinisch erprobtes Antiepileptikum mit antikonvulsiver Wirkung. Gabapentin zeigt zudem weitere interessante, medizinische relevante Eigenschaften, insbesondere als Analgetikum. Gabapentin wird von behandelnden Ärzten auch bei Migräne und bipolaren Störungen sowie Hitzewallungen (z.B. in der Postmenopause) verschrieben (M. Schrope, Modern Drug Discovery, September 2000, S. 11). Andere Indikationenen, in denen Gabapentin ein therapeutisches Potential zeigt, wurden während der Humanstudien und im klinischen Gebrauch identifiziert (J.S. Bryans, D.J. Wustrow; "3-Substituted GABA Analogs with Central Nervous System Activity: A Review" in Med. Res. Rev. (1999), S. 149-177). In diesem Übersichtsartikel wird detailliert die Wirkung von Gabapentin aufgelistet. So ist Gabapentin wirksam in der Behandlung chronischer Schmerzen und Verhaltensstörungen. Insbesondere sind aufgeführt: Antikonvulsive und antiepileptische Wirkungen, der Einsatz gegen chronischen, neuropathischen Schmerz, insbesondere thermische Hyperalgesie, mechanische Allodynie, Kälte-Allodynie. Weiter wirkt es gegen durch Nervenschädigungen ausgelöste Neuropathie, insbesondere eben neuropathischen Schmerz, wie auch inflammatorischen und postoperativen Schmerz erfolgreich. Gabapentin ist auch erfolgreich bei antipsychotischen Effekten insbesondere als Anxiolytikum. Weitere überprüfte Indikationen umfassen: Amyotropische Laterale Sklerose (ALS), Reflex Sympasthetic Dystrophy (RSD), Spastische Lähmung, Restless Leg Syndrom, Behandlung von Symptomen und Schmerz aufgrund von Multipler Sklerose, erworbener Nystagmus, Behandlung der Symptome der Parkinsonschen Krankheit, der schmerzvollen diabetischen Neuropathie und psychatrischer Störungen, z.B. bipolare Störungen, Stimmungsschwankungen, manisches Verhalten. Weiter erfolgreich war der Einsatz von Gabapentin bei erythromelalgischem Schmerz, postpoliomyelitisem Schmerz, trigeminaler Neuralgie und postherpetischer Neuralgie (Bryans und Wustrow (1999), a.a.O.). Allgemein bekannt und auch dem genannten Übersichtsartikel anhand der Beispiele zu entnehmen, ist auch die allgemeine Wirksamkeit in neurodegenerativen Erkrankungen. Solche Neurodegenerativen Erkrankungen sind z.B. Morbus Alzheimer, Morbus Huntington, Morbus Parkinson und Epilepsie. Bekannt ist auch die Wirksamkeit von Gabapentin bei gastrointestinalen Schädigungen.

Aufgabe der Erfindung war es daher, neue Arzneimittel, die in ihren Wirkung Übereinstimmungen mit Gabapentin zeigen und vorzugsweise analgetische und/oder antiepileptischen Wirkung besitzen, zur Verfügung zu stellen.

Erfindungsgemäß wird diese Aufgabe durch Bereitstellung der Arzneimittel enthaltend N,N'-disubstituierte Piperazin-Verbindungen der nachstehend angegebenen Formel I gelöst, da diese Arzneimittel insbesondere eine ausgeprägte analgetische Wirkung und eine ausgeprägte Wirkung gegen Epilepsie aufweisen und zur Behandlung von neuropathischem, chronischem und/oder akutem Schmerz, zur Behandlung insbesondere mit neuropathischem Schmerz verbundener Symptome, zur Behandlung von Migräne, Hyperalgesie und/oder Allodynie, insbesondere thermischer Hyperalgesie, mechanischer Hyperalgesie, mechanischer Allodynie und/oder Kälte-Allodynie, zur Behandlung von inflammatorischem und/oder postoperativem Schmerz, zur Behandlung von neurodegenerativen Erkrankungen, wie Morbus Alzheimer, Morbus Huntington, Morbus Parkinson und/oder Epilepsie, zur Behandlung von psychatrischen und/oder neuropathologischen Störungen, wie bipolaren Störungen, Anxiety, Panikanfällen, Stimmungsschwankungen, manischem Verhalten, manisch-depressivem Verhalten, zur Behandlung von Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus, schmerzvoller diabetischer Neuropathie, zur Behandlung von Symptomen und/oder Schmerzen aufgrund von Multipler Sklerose und/oder der Parkinsonschen Krankheit, zur Behandlung von gastrointestinaler Schädigung, erythromelalgischem und/oder postpoliomyelitischem Schmerz, trigeminaler und/oder postherpetischer Neuralgie eingesetzt werden können.

Gegenstand der Erfindung ist daher die Verwendung wenigstens eine N,N'-disubstituierten Piperazin-Verbindung der allgemeinen Formel I, 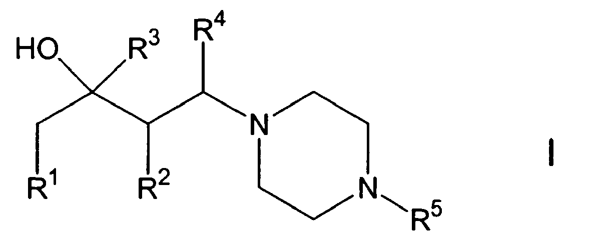 worin
R¹ und R², gleich oder verschieden, für jeweils einen linearen oder verzweigten, gesättigten oder ungesättigten aliphatischen Rest stehen oder zusammen eine (CH₂)ₙ-Kette bilden, wobei n für eine ganze Zahl steht,
R³ und R⁵, gleich oder verschieden, für jeweils einen linearen oder verzweigten, gesättigten oder ungesättigten aliphatischen Rest, einen gesättigten oder ungesättigten cycloaliphatischen Rest, einen Arylrest oder einen Heteroarylrest stehen, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann,
R⁴ für Wasserstoff oder einen gegebenenfalls einfach oder mehrfach substituierten Aryl- oder Heteroarylrest steht, wobei der Aryl- oder Heteroarylrest Teil eines polycyclischen Systems sein kann,
in Form ihrer Diastereomere, ihrer Enantiomere und Gemische davon - einschließlich ihrer Racemate - sowie in Form entsprechender Basen, Salze und Solvate als Wirkstoff
zur Herstellung eines Arzneimittels zur Behandlung von
- Epilepsie,
- neuropathischem, chronischem und/oder akutem Schmerz,
- mit neuropathischem Schmerz verbundener Symptome
- Migräne,
- Hyperalgesie, insbesondere thermischer Hyperalgesie und/oder mechanischer Hyperalgesie,
- Allodynie, insbesondere mechanischer Allodynie und/oder Kälte-Allodynie,
- inflammatorischem Schmerz,
- postoperativem Schmerz,
- neurodegenerativen Erkrankungen, insbesondere Morbus Alzheimer, Morbus Huntington und/oder Morbus Parkinson,
- psychiatrischen Störungen und/oder neuropathologischen Störungen, insbesondere bipolaren Störungen, Anxiety, Panikanfällen, Stimmungsschwankungen, manischem Verhalten und/oder manisch-depressivem Verhalten,
- Hitzewallungen,
- Beschwerden in der Postmenopause,
- Amyotropischer Lateraler Sklerose (ALS),
- Reflex Sympasthetic Dystrophy (RSD),
- Spastischer Lähmung,
- Restless Leg Syndrome,
- erworbenem Nystagmus,
- schmerzvoller diabetischer Neuropathie,
- Symptomen und/oder Schmerzen aufgrund von Multipler Sklerose und/oder der Parkinsonschen Krankheit,
- gastrointestinaler Schädigung,
- erythromelalgischem Schmerz,
- postpoliomyelitischem Schmerz,
- trigeminaler Neuralgie und/oder
- postherpetischer Neuralgie.

Bevorzugt ist die Verwendung wenigstens einer Verbindung der allgemeinen Formel I, worin
R¹ und R², gleich oder verschieden, für jeweils einen linearen oder verzweigten, gesättigten oder ungesättigten aliphatischen C₁₋₆-Rest stehen oder zusammen eine (CH₂)ₙ-Kette bilden, wobei n für eine ganze Zahl von 2-9 steht,
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann,
R⁴ für Wasserstoff oder einen Phenylrest steht,
R⁵ für einen linearen oder verzweigten, gesättigten oder ungesättigten aliphatischen C₁₋₆-Rest oder einen gegebenenfalls substituierten Phenylrest steht.

Weiter bevorzugt ist die Verwendung wenigstens einer Verbindung der allgemeinen Formel I, worin

R¹ und R², gleich oder verschieden, für jeweils einen linearen oder verzweigten, gesättigten oder ungesättigten aliphatischen C₁₋₃-Rest stehen oder zusammen eine (CH₂)ₙ-Kette bilden, wobei n für eine ganze Zahl von 2 - 9 steht,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁₋₃-Brücke gebunden sein kann,
R⁴ für Wasserstoff oder einen Phenylrest steht,
R⁵ für einen linearen oder verzweigten, gesättigten oder ungesättigten aliphatischen C₁₋₃-Rest oder einen gegebenenfalls mit Halogen oder einer Alkoxygruppe substituierten Phenylrest steht.

Weiter bevorzugt ist die Verwendung wenigstens einerVerbindung der allgemeinen Formel I, worin
R¹ und R² jeweils für eine Methylgruppe stehen oder zusammen eine (CH₂)ₙ-Kette bilden, wobei n für 3, 4, 5 oder 9 steht,
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann,
R⁴ für Wasserstoff oder einen Phenylrest steht,
R⁵ für eine Methylgruppe oder einen gegebenenfalls mit Chlor oder einer Methoxygruppe substituierten Phenylrest steht.

Besonders bevorzugt ist die Verwendung wenigstens einer Verbindung der allgemeinen Formel II, 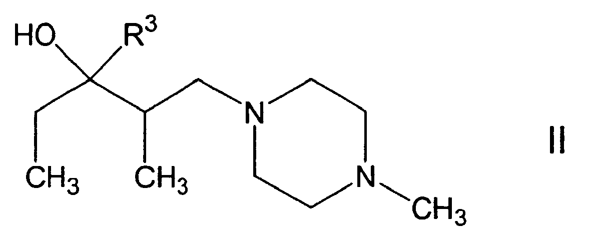 worin
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt ist die Verwendung wenigstens einer Verbindung der allgemeinen Formel III, 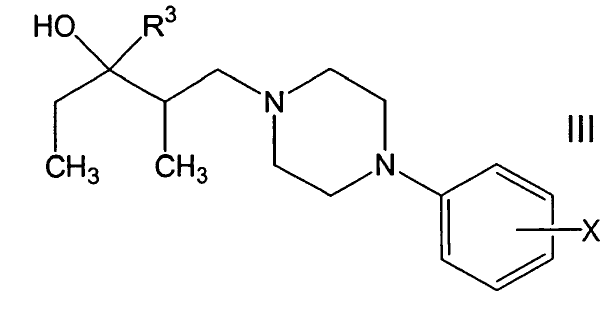 worin
X für Wasserstoff, Halogen oder eine Alkoxygruppe, vorzugsweise für Wasserstoff, Chlor oder eine Methoxygruppe, steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt ist die Verwendung wenigstens einer Verbindung der allgemeinen Formel IV, 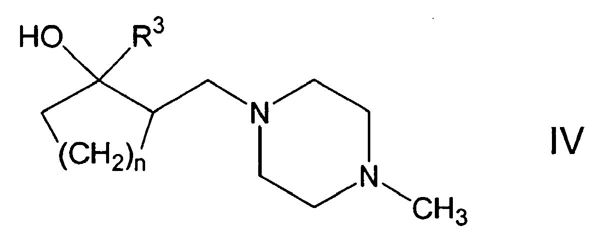 worin
n für eine ganze Zahl von 2-9 steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt ist die Verwendung wenigstens einer Verbindung der allgemeinen Formel V, 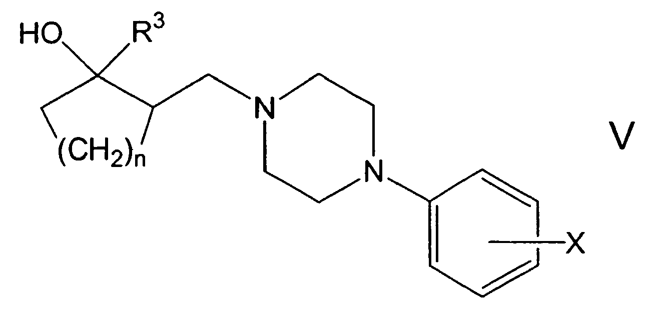 worin
n für eine ganze Zahl von 2 - 9 steht,
X für Wasserstoff, Halogen oder eine Alkoxygruppe, vorzugsweise für Wasserstoff, Chlor oder eine Methoxygruppe, steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt ist die Verwendung wenigstens einer Verbindung der allgemeinen Formel VI, 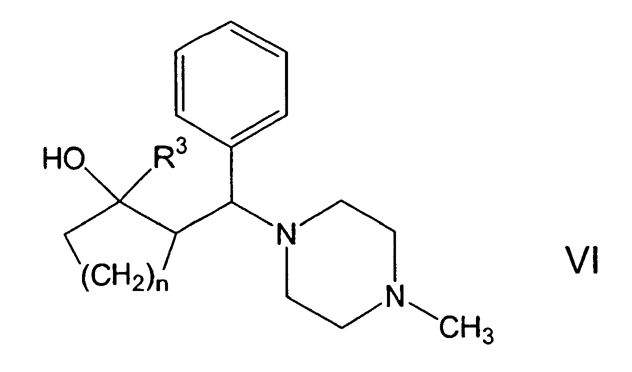 worin
n für eine ganze Zahl von 2 - 9 steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt ist die Verwendung wenigstens einer Verbindung der allgemeinen Formel VII, 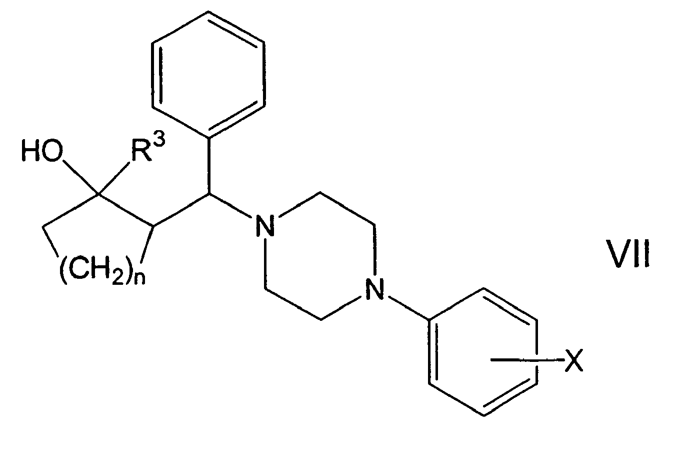 worin
n für eine ganze Zahl von 2 - 9 steht,
X für Wasserstoff, Halogen oder eine Alkoxygruppe, vorzugsweise für Wasserstoff, Chlor oder eine Methoxygruppe, steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Weiter bevorzugt ist die Verwendung wenigstens einer Verbindung der allgemeinen Formel II-VII, worin
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann
und die übrigen Symbole die vorstehend genannte Bedeutung haben.

Weiter bevorzugt ist die Verwendung wenigstens einer Verbindung der allgemeinen Formel II-VII, worin
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁₋₃-Brücke gebunden sein kann
und die übrigen Symbole die vorstehend genannte Bedeutung haben.

Weiter bevorzugt ist die Verwendung wenigstens einer Verbindung der allgemeinen Formel II-VII, worin
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann
und die übrigen Symbole die vorstehend genannte Bedeutung haben.

Unter einem Heteroarylrest wird ein gegebenenfalls ein- oder mehrfach substituierter, fünf- oder sechsgliedriger aromatischer Rest mit mindestens einem, gegebenenfalls 2, 3, 4 oder 5 Heteroatomen, die gleich oder verschieden sein können, verstanden, der Teil eines polycylischen Systems sein kann. Bevorzugte Heteroatome sind Stickstoff, Sauerstoff und Schwefel. Besonders bevorzugt sind Heteroarylreste ausgewählt aus der Gruppe umfassend Pyrrolyl-, Indolyl-, Furyl- (Furanyl-), Benzofuranyl-, Thienyl- (Thiophenyl-), Benzothienyl-, Pyrazolyl-, Imidazolyl-, Thiazolyl-, Oxazolyl-, lsoxazoyl-, Pydridinyl-, Pyridazinyl-, Pyrimidinyl-, Pyrazinyl-, Pyranyl-, Indazolyl-, Purinyl-, Indolizinyl-, Chinolinyl-, lsochinolinyl-, Chinazolinyl-, Carbazolyl-, Phenazinyl- und Phenothiazinylrest. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Unter einem Arylrest wird ein gegebenenfalls ein- oder mehrfach substituierter, aromatischer Rest verstanden, der Teil eines polycylischen Systems sein kann. Besonders bevorzugt ist ein Phenylrest. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Ganz besonders bevorzugt ist die Verwendung wenigstens einer Verbindung ausgewählt aus der Gruppe umfassend
2-Methyl-1-(4-methyl-piperazin-1-yl)-3-phenyl-pentan-3-ol
3-(4-Chlor-phenyl)-2-methyl-1-(4-methyl-piperazin-1-yl)-pentan-3-ol
3-Benzyl-2-methyl-1-(4-methyl-piperazin-1-yl)-pentan-3-ol
3-(4-Fluor-3-methyl-phenyl)-2-methyl-1-(4-methyl-piperazin-1-yl)-pentan-3-ol
2-Methyl-1-(4-methyl-piperazin-1-yl)-3-o-tolyl-pentan-3-ol
3-Ethyl-4-methyl-5-(4-methyl-piperazin-1-yl)-pent-1-en-3-ol
3-(4-tert-Butyl-phenyl)-2-methyl-1-(4-methyl-piperazin-1-yl)-pentan-3-ol
3-Cyclopentyl-2-methyl-1-(4-methyl-piperazin-1-yl)-pentan-3-ol
2-Methyl-1-(4-methyl-piperazin-1-yl)-3-m-tolyl-pentan-3-ol
3-Cyclohexyl-2-methyl-1-(4-methyl-piperazin-1-yl)-pentan-3-ol
3-(4-Fluor-phenyl)-2-methyl-1-(4-methyl-piperazin-1-yl)-pentan-3-ol
3-Ethyl-2-methyl-1-(4-methyl-piperazin-1-yl)-5-phenyl-pentan-3-ol
3-Ethyl-4-methyl-5-(4-methyl-piperazin-1-yl)-1-phenyl-pent-1-yn-3-ol
2-Methyl-1-(4-methyl-piperazin-1-yl)-3-thiophen-2-yl-pentan-3-ol
3-(3-Methoxy-phenyl)-2-methyl-1-(4-methyl-piperazin-1-yl)-pentan-3-ol
3-Ethyl-2-methyl-1-(4-methyl-piperazin-1-yl)-6-phenyl-hexan-3-ol
2-Methyl-1-(4-methyl-piperazin-1-yl)-3-p-tolyl-pentan-3-ol
3-(4-Methoxy-phenyl)-2-methyl-1-(4-methyl-piperazin-1-yl)-pentan-3-ol
2-(4-Methyl-piperazin-1-ylmethyl)-1-phenyl-cyclohexanol
1-Benzyl-2-(4-methyl-piperazin-1-ylmethyl)-cyclohexanol
1-(4-Fluor-3-methyl-phenyl)-2-(4-methyl-piperazin-1-ylmethyl)-cyclohexanol
2-(4-Methyl-piperazin-1-ylmethyl)-1-o-tolyl-cyclohexanol
2-(4-Methyl-piperazin-1-ylmethyl)-1-vinyl-cyclohexanol
1-Cyclopentyl-2-(4-methyl-piperazin-1-ylmethyl)-cyclohexanol
2-(4-Methyl-piperazin-1-ylmethyl)-1-m-tolyl-cyclohexanol
2-(4-Methyl-piperazin-1-ylmethyl)-bicyclohexyl-1-ol
1-(4-Fluor-phenyl)-2-(4-methyl-piperazin-1-ylmethyl)-cyclohexanol
2-(4-Methyl-piperazin-1 -ylmethyl)-1 -phenethyl-cyclohexanol
2-(4-Methyl-piperazin-1-ylmethyl)-1-thiophen-2-yl-cyclohexanol
1-(2,4-Dichlor-phenyl)-2-(4-methyl-piperazin-1-ylmethyl)-cyclohexanol
1-(3-Methoxy-phenyl)-2-(4-methyl-piperazin-1-ylmethyl)-cyclohexanol
2-(4-Methyl-piperazin-1 -ylmethyl)-1 -(3-phenyl-propyl)-cyclohexanol
2-(4-Methyl-piperazin-1-ylmethyl)-1-p-tolyl-cyclohexanol
1-(4-Methoxy-phenyl)-2-(4-methyl-piperazin-1-ylmethyl)-cyclohexanol
1 -Benzyl-2-(4-methyl-piperazin-1 -ylmethyl)-cyclooctanol
2-(4-Methyl-piperazin-1-ylmethyl)-1-vinyl-cyclooctanol
1-(4-tert-Butyl-phenyl)-2-(4-methyl-piperazin-1-ylmethyl)-cyclooctanol
1-Cyclopentyl-2-(4-methyl-piperazin-1-ylmethyl)-cyclooctanol
2-(4-Methyl-piperazin-1 -ylmethyl)-1 -phenethyl-cyclooctanol
2-(4-Methyl-piperazin-1-ylmethyl)-1-phenylethynyl-cyclooctanol
2-(4-Methyl-piperazin-1-ylmethyl)-1-thiophen-2-yl-cyclooctanol
1-(3-Methoxy-phenyl)-2-(4-methyl-piperazin-1-ylmethyl)-cyclooctanol
2-(4-Methyl-piperazin-1-ylmethyl)-1-(3-phenyl-propyl)-cyclooctanol
2-(4-Methyl-piperazin-1-ylmethyl)-1-p-tolyl-cyclooctanol
1-(4-Methoxy-phenyl)-2-(4-methyl-piperazin-1-ylmethyl)-cyclooctanol
2-(4-Methyl-piperazin-1-ylmethyl)-1-phenyl-cycloheptanol
1-(4-Chlor-phenyl)-2-(4-methyl-piperazin-1-ylmethyl)-cycloheptanol
1-Benzyl-2-(4-methyl-piperazin-1-ylmethyl)-cycloheptanol
1-(4-Fluor-3-methyl-phenyl)-2-(4-methyl-piperazin-1-ylmethyl)-cycloheptanol
2-(4-Methyl-piperazin-1-ylmethyl)-1-o-tolyl-cycloheptanol
2-(4-Methyl-piperazin-1 -ylmethyl)-1 -vinyl-cycloheptanol
1-(4-tert-Butyl-phenyl)-2-(4-methyl-piperazin-1-ylmethyl)-cycloheptanol
1-Cyclopentyl-2-(4-methyl-piperazin-1-ylmethyl)-cycloheptanol
2-(4-Methyl-piperazin-1-ylmethyl)-1-m-tolyl-cycloheptanol
1-[4-(3-Chlor-phenyl)-piperazin-1-yl]-2-methyl-3-phenyl-pentan-3-ol
3-(4-Chlor-phenyl)-1-[4-(3-chlor-phenyl)-piperazin-1-yl]-2-methyl-pentan-3-ol
3-Benzyl-1-[4-(3-chlor-phenyl)-piperazin-1-yl]-2-methyl-pentan-3-ol
1-[4-(3-Chlor-phenyl)-piperazin-1-yl]-3-(4-fluor-3-methyl-phenyl)-2-methyl-pentan-3-ol
5-[4-(3-Chlor-phenyl)-piperazin-1-yl]-3-ethyl-4-methyl-pent-1-en-3-ol
3-(4-tert-Butyl-phenyl)-1-[4-(3-chlor-phenyl)-piperazin-1-yl]-2-methyl-pentan-3-ol
1-[4-(3-Chlor-phenyl)-piperazin-1-yl]-2-methyl-3-m-tolyl-pentan-3-ol
1-[4-(3-Chlor-phenyl)-piperazin-1-yl]-3-cyclohexyl-2-methyl-pentan-3-ol
1-[4-(3-Chlor-phenyl)-piperazin-1-yl]-3-(4-fluor-phenyl)-2-methyl-pentan-3-ol
1-[4-(3-Chlor-phenyl)-piperazin-1-yl]-3-ethyl-2-methyl-5-phenyl-pentan-3-ol
5-[4-(3-Chlor-phenyl)-piperazin-1-yl]-3-ethyl-4-methyl-1-phenyl-pent-1-yn-3-ol
1-[4-(3-Chlor-phenyl)-piperazin-1-yl]-2-methyl-3-thiophen-2-yl-pentan-3-ol
1-[4-(3-Chlor-phenyl)-piperazin-1-yl]-3-(3-methoxy-phenyl)-2-methyl-pentan-3-ol
1-[4-(3-Chlor-phenyl)-piperazin-1-yl]-3-ethyl-2-methyl-6-phenyl-hexan-3-ol
1-[4-(3-Chlor-phenyl)-piperazin-1-yl]-2-methyl-3-p-tolyl-pentan-3-ol
1-[4-(3-Chlor-phenyl)-piperazin-1-yl]-3-(4-methoxy-phenyl)-2-methyl-pentan-3-ol
1-[4-(3-Chlor-phenyl)-piperazin-1-yl]-3-(5-fluor-2-methoxy-phenyl)-2-methyl-pentan-3-ol
1-[4-(3-Chlor-phenyl)-piperazin-1-yl]-3-(3-fluor-phenyl)-2-methyl-pentan-3-ol
3-(3-Chlor-phenyl)-1-[4-(3-chlor-phenyl)-piperazin-1-yl]-2-methyl-pentan-3-ol
3-(2-Chlor-benzyl)-1-[4-(3-chlor-phenyl)-piperazin-1-yl]-2-methyl-pentan-3-ol
1-[4-(3-Chlor-phenyl)-piperazin-1-yl]-3-(4-fluor-benzyl)-2-methyl-pentan-3-ol
1-[4-(3-Chlor-phenyl)-piperazin-1-yl]-3-(3-methoxy-benzyl)-2-methyl-pentan-3-ol
1-[4-(3-Chlor-phenyl)-piperazin-1-yl]-3-(3-fluor-benzyl)-2-methyl-pentan-3-ol
1-[4-(3-Chlor-phenyl)-piperazin-1-yl]-3-(2-methoxy-phenyl)-2-methyl-pentan-3-ol
1-[4-(3-Chlor-phenyl)-piperazin-1-yl]-2-methyl-3-(2-methyl-benzyl)-pentan-3-ol
1-[4-(3-Chlor-phenyl)-piperazin-1-yl]-2-methyl-3-(3-trifluormethyl-phenyl)-pentan-3-ol
1-[4-(3-Chlor-phenyl)-piperazin-1-yl]-2-methyl-3-(3-methyl-benzyl)-pentan-3-ol
3-(4-Chlor-benzyl)-1-[4-(3-chlor-phenyl)-piperazin-1-yl]-2-methyl-pentan-3-ol
3-(2-Chlor-6-fluor-benzyl)-1-[4-(3-chlor-phenyl)-piperazin-1-yl]-2-methyl-pentan-3-ol
1-[4-(3-Chlor-phenyl)-piperazin-1-yl]-3-(2,5-dimethyl-benzyl)-2-methyl-pentan-3-ol
3-(3-Chlor-benzyl)-1-[4-(3-chlor-phenyl)-piperazin-1-yl]-2-methyl-pentan-3-ol
1-[4-(3-Chlor-phenyl)-piperazin-1-yl]-3-(2,4-dichlor-benzyl)-2-methyl-pentan-3-ol
3-Cyclohexylmethyl-2-methyl-1-(4-methyl-piperazin-1-yl)-pentan-3-ol
3-(5-Fluor-2-methoxy-phenyl)-2-methyl-1-(4-methyl-piperazin-1-yl)-pentan-3-ol
3-(3-Fluor-phenyl)-2-methyl-1-(4-methyl-piperazin-1-yl)-pentan-3-ol
3-(3-Chlor-phenyl)-2-methyl-1-(4-methyl-piperazin-1-yl)-pentan-3-ol
3-(3,5-Dichlor-phenyl)-2-methyl-1-(4-methyl-piperazin-1-yl)-pentan-3-ol
3-(2-Chlor-benzyl)-2-methyl-1-(4-methyl-piperazin-1-yl)-pentan-3-ol
3-(4-Fluor-benzyl)-2-methyl-1-(4-methyl-piperazin-1-yl)-pentan-3-ol
3-(3-Methoxy-benzyl)-2-methyl-1-(4-methyl-piperazin-1-yl)-pentan-3-ol
3-(4-Chlor-3-trifluormethyl-phenyl)-2-methyl-1-(4-methyl-piperazin-1-yl)-pentan-3-ol
3-(3-Fluor-benzyl)-2-methyl-1-(4-methyl-piperazin-1-yl)-pentan-3-ol
3-(2-Methoxy-phenyl)-2-methyl-1-(4-methyl-piperazin-1-yl)-pentan-3-ol
2-Methyl-3-(2-methyl-benzyl)-1-(4-methyl-piperazin-1-yl)-pentan-3-ol
3-(3-Chlor-4-fluor-phenyl)-2-methyl-1-(4-methyl-piperazin-1-yl)-pentan-3-ol
2-Methyl-1-(4-methyl-piperazin-1-yl)-3-(3-trifluormethyl-phenyl)-pentan-3-ol
2-Methyl-3-(3-methyl-benzyl)-1-(4-methyl-piperazin-1-yl)-pentan-3-ol
3-(4-Chlor-benzyl)-2-methyl-1-(4-methyl-piperazin-1-yl)-pentan-3-ol
3-(2-Chlor-6-fluor-benzyl)-2-methyl-1-(4-methyl-piperazin-1-yl)-pentan-3-ol
3-(2,5-Dimethyl-benzyl)-2-methyl-1-(4-methyl-piperazin-1-yl)-pentan-3-ol
3-(3-Chlor-benzyl)-2-methyl-1-(4-methyl-piperazin-1-yl)-pentan-3-ol
3-(2,4-Dichlor-benzyl)-2-methyl-1-(4-methyl-piperazin-1-yl)-pentan-3-ol
1-Cyclohexylmethyl-2-(4-methyl-piperazin-1-ylmethyl)-cyclohexanol
1-(5-Fluor-2-methoxy-phenyl)-2-(4-methyl-piperazin-1-ylmethyl)-cyclohexanol
1-(3-Fluor-phenyl)-2-(4-methyl-piperazin-1-ylmethyl)-cyclohexanol
1-(3-Chlor-phenyl)-2-(4-methyl-piperazin-1-ylmethyl)-cyclohexanol
1-(3,5-Dichlor-phenyl)-2-(4-methyl-piperazin-1-ylmethyl)-cyclohexanol
1-(2-Chlor-benzyl)-2-(4-methyl-piperazin-1-ylmethyl)-cyclohexanol
1-(4-Fluor-benzyl)-2-(4-methyl-piperazin-1-ylmethyl)-cyclohexanol
1-(3-Methoxy-benzyl)-2-(4-methyl-piperazin-1-ylmethyl)-cyclohexanol
1-(4-Chlor-3-trifluormethyl-phenyl)-2-(4-methyl-piperazin-1-ylmethyl)-cyclohexanol
1-(3-Fluor-benzyl)-2-(4-methyl-piperazin-1-ylmethyl)-cyclohexanol
1-(2-Methoxy-phenyl)-2-(4-methyl-piperazin-1-ylmethyl)-cyclohexanol
1-(2-Methyl-benzyl)-2-(4-methyl-piperazin-1-ylmethyl)-cyclohexanol
1-(3-Chlor-4-fluor-phenyl)-2-(4-methyl-piperazin-1-ylmethyl)-cyclohexanol
2-(4-Methyl-piperazin-1-ylmethyl)-1-(3-trifluormethyl-phenyl)-cyclohexanol
1-(3-Methyl-benzyl)-2-(4-methyl-piperazin-1-ylmethyl)-cyclohexanol
1-(4-Chlor-benzyl)-2-(4-methyl-piperazin-1-ylmethyl)-cyclohexanol
1-(2,5-Dimethyl-benzyl)-2-(4-methyl-piperazin-1-ylmethyl)-cyclohexanol
1-(3-Chlor-benzyl)-2-(4-methyl-piperazin-1-ylmethyl)-cyclohexanol
1-(2,4-Dichlor-benzyl)-2-(4-methyl-piperazin-1-ylmethyl)-cyclohexanol 1-Cyclohexylmethyl-2-(4-methyl-piperazin-1-ylmethyl)-cyclooctanol
1-(5-Fluor-2-methoxy-phenyl)-2-(4-methyl-piperazin-1-ylmethyl)-cyclooctanol
1-(2-Chlor-benzyl)-2-(4-methyl-piperazin-1-ylmethyl)-cyclooctanol
1-(4-Fluor-benzyl)-2-(4-methyl-piperazin-1-ylmethyl)-cyclooctanol
1-(3-Fluor-benzyl)-2-(4-methyl-piperazin-1-ylmethyl)-cyclooctanol
1-(2-Methoxy-phenyl)-2-(4-methyl-piperazin-1-ylmethyl)-cyclooctanol
1-(3-Methyl-benzyl)-2-(4-methyl-piperazin-1-ylmethyl)-cyclooctanol
1-(4-Chlor-benzyl)-2-(4-methyl-piperazin-1-ylmethyl)-cyclooctanol
1-(2-Chlor-6-fluor-benzyl)-2-(4-methyl-piperazin-1-ylmethyl)-cyclooctanol
1-(2,5-Dimethyl-benzyl)-2-(4-methyl-piperazin-1-ylmethyl)-cyclooctanol
1-(3-Chlor-benzyl)-2-(4-methyl-piperazin-1-ylmethyl)-cyclooctanol
1-(2,4-Dichlor-benzyl)-2-(4-methyl-piperazin-1-ylmethyl)-cyclooctanol
1-Cyclohexylmethyl-2-(4-methyl-piperazin-1-ylmethyl)-cycloheptanol
1-(5-Fluor-2-methoxy-phenyl)-2-(4-methyl-piperazin-1-ylmethyl)-cycloheptanol
1-(3-Fluor-phenyl)-2-(4-methyl-piperazin-1-ylmethyl)-cycloheptanol
1-(3-Chlor-phenyl)-2-(4-methyl-piperazin-1-ylmethyl)-cycloheptanol
1-(3,5-Dichlor-phenyl)-2-(4-methyl-piperazin-1-ylmethyl)-cycloheptanol
1-(2-Chlor-benzyl)-2-(4-methyl-piperazin-1-ylmethyl)-cycloheptanol
1-(4-Fluor-benzyl)-2-(4-methyl-piperazin-1-ylmethyl)-cycloheptanol
1-(3-Methoxy-benzyl)-2-(4-methyl-piperazin-1-ylmethyl)-cycloheptanol
1-(4-Chlor-3-trifluormethyl-phenyl)-2-(4-methyl-piperazin-1-ylmethyl)-cycloheptanol
1-(3-Fluor-benzyl)-2-(4-methyl-piperazin-1-ylmethyl)-cycloheptanol
1-(2-Methoxy-phenyl)-2-(4-methyl-piperazin-1-ylmethyl)-cycloheptanol
1-(2-Methyl-benzyl)-2-(4-methyl-piperazin-1-ylmethyl)-cycloheptanol
1-(3-Chlor-4-fluor-phenyl)-2-(4-methyl-piperazin-1-ylmethyl)-cycloheptanol
2-(4-Methyl-piperazin-1-ylmethyl)-1-(3-trifluormethyl-phenyl)-cycloheptanol
1-(3-Methyl-benzyl)-2-(4-methyl-piperazin-1-ylmethyl)-cycloheptanol
1-(4-Chlor-benzyl)-2-(4-methyl-piperazin-1-ylmethyl)-cycloheptanol
1-(2-Chlor-6-fluor-benzyl)-2-(4-methyl-piperazin-1-ylmethyl)-cycloheptanol
1-(2,5-Dimethyl-benzyl)-2-(4-methyl-piperazin-1-ylmethyl)-cycloheptanol
1-(3-Chlor-benzyl)-2-(4-methyl-piperazin-1-ylmethyl)-cycloheptanol
1-(2,4-Dichlor-benzyl)-2-(4-methyl-piperazin-1-ylmethyl)-cycloheptanol
1-Benzyl-2-(4-methyl-piperazin-1-ylmethyl)-cyclododecanol
2-(4-Methyl-piperazin-1 -ylmethyl)-1 -vinyl-cyclododecanol
1-Cyclopentyl-2-(4-methyl-piperazin-1-ylmethyl)-cyclododecanol
2-(4-Methyl-piperazin-1-ylmethyl)-1-phenethyl-cyclododecanol
2-(4-Methyl-piperazin-1-ylmethyl)-1-phenylethynyl-cyclododecanol
2-(4-Methyl-piperazin-1-ylmethyl)-1-thiophen-2-yl-cyclododecanol
2-(4-Methyl-piperazin-1-ylmethyl)-1-(3-phenyl-propyl)-cyclododecanol
1-(5-Fluor-2-methoxy-phenyl)-2-(4-methyl-piperazin-1-ylmethyl)-cyclododecanol
1-(2-Chlor-benzyl)-2-(4-methyl-piperazin-1-ylmethyl)-cyclododecanol
1-(4-Fluor-benzyl)-2-(4-methyl-piperazin-1-ylmethyl)-cyclododecanol
1-(3-Methoxy-benzyl)-2-(4-methyl-piperazin-1-ylmethyl)-cyclododecanol
1-(3-Fluor-benzyl)-2-(4-methyl-piperazin-1-ylmethyl)-cyclododecanol
1-(2-Methoxy-phenyl)-2-(4-methyl-piperazin-1-ylmethyl)-cyclododecanol
1-(2-Methyl-benzyl)-2-(4-methyl-piperazin-1-ylmethyl)-cyclododecanol
1-(3-Methyl-benzyl)-2-(4-methyl-piperazin-1-ylmethyl)-cyclododecanol
1-(4-Chlor-benzyl)-2-(4-methyl-piperazin-1-ylmethyl)-cyclododecanol
1-(2,5-Dimethyl-benzyl)-2-(4-methyl-piperazin-1-ylmethyl)-cyclododecanol
1-(3-Chlor-benzyl)-2-(4-methyl-piperazin-1-ylmethyl)-cyclododecanol
1-(2,4-Dichlor-benzyl)-2-(4-methyl-piperazin-1-ylmethyl)-cyclododecanol
2-[4-(3-Chlor-phenyl)-piperazin-1-ylmethyl]-1-cyclohexylmethyl-cyclohexanol
2-[4-(3-Chlor-phenyl)-piperazin-1-ylmethyl]-1-(5-fluor-2-methoxy-phenyl)-cyclohexanol
2-[4-(3-Chlor-phenyl)-piperazin-1-ylmethyl]-1-(3-fluor-phenyl)-cyclohexanol
1-(3-Chlor-phenyl)-2-[4-(3-chlor-phenyl)-piperazin-1 -ylmethyl]-cyclohexanol
2-[4-(3-Chlor-phenyl)-piperazin-1-ylmethyl]-1-(3,5-dichlor-phenyl)-cyclohexanol
1-(2-Chlor-benzyl)-2-[4-(3-chlor-phenyl)-piperazin-1-ylmethyl]-cyclohexanol
2-[4-(3-Chlor-phenyl)-piperazin-1-ylmethyl]-1-(4-fluor-benzyl)-cyclohexanol
2-[4-(3-Chlor-phenyl)-piperazin-1-ylmethyl]-1-(4-chlor-3-trifluormethyl-phenyl)-cyclohexanol
2-[4-(3-Chlor-phenyl)-piperazin-1-ylmethyl]-1-(3-fluor-benzyl)-cyclohexanol
2-[4-(3-Chlor-phenyl)-piperazin-1-ylmethyl]-1-(2-methoxy-phenyl)-cyclohexanol
1-(3-Chlor-4-fluor-phenyl)-2-[4-(3-chlor-phenyl)-piperazin-1-ylmethyl]-cyclohexanol
2-[4-(3-Chlor -phenyl)-piperazin-1-ylmethyl]-1-(3-trifluormethyl-phenyl)-cyclohexanol
2-[4-(3-Chlor-phenyl)-piperazin-1-ylmethyl]-1-(3-methyl-benzyl)-cyclohexanol
1-(4-Chlor-benzyl)-2-[4-(3-chlor-phenyl)-piperazin-1-ylmethyl]-cyclohexanol
1-(2-Chlor-6-fluor-benzyl)-2-[4-(3-chlor-phenyl)-piperazin-1-ylmethyl]-cyclohexanol 2-[4-(3-Chlor-phenyl)-piperazin-1-ylmethyl]-1-(2,5-dimethyl-benzyl)-cyclohexanol
1-(3-Chlor-benzyl)-2-[4-(3-chlor-phenyl)-piperazin-1-ylmethyl]-cyclohexanol
2-[4-(2-Chlor-phenyl)-piperazin-1-ylmethyl]-1-phenyl-cyclohexanol
1-(4-Chlor-phenyl)-2-[4-(2-chlor-phenyl)-piperazin-1-ylmethyl]-cyclohexanol
1-Benzyl-2-[4-(2-chlor-phenyl)-piperazin-1-ylmethyl]-cyclohexanol
2-[4-(2-Chlor-phenyl)-piperazin-1-ylmethyl]-1-(4-fluor-3-methyl-phenyl)-cyclohexanol
2-[4-(2-Chlor-phenyl)-piperazin-1-ylmethyl]-1-o-tolyl-cyclohexanol
2-[4-(2-Chlor-phenyl)-piperazin-1-ylmethyl]-1-vinyl-cyclohexanol
1-(4-tert-Butyl-phenyl)-2-[4-(2-chlor-phenyl)-piperazin-1-ylmethyl]-cyclohexanol
2-[4-(2-Chlor-phenyl)-piperazin-1-ylmethyl]-1-cyclopentyl-cyclohexanol
2-[4-(2-Chlor-phenyl)-piperazin-1-ylmethyl]-1-m-tolyl-cyclohexanol
2-[4-(2-Chlor-phenyl)-piperazin-1-ylmethyl]-bicyclohexyl-1-ol
2-[4-(2-Chlor-phenyl)-piperazin-1-ylmethyl]-1-(4-fluor-phenyl)-cyclohexanol
2-[4-(2-Chlor-phenyl)-piperazin-1-ylmethyl]-1-phenethyl-cyclohexanol
2-[4-(2-Chlor-phenyl)-piperazin-1-ylmethyl]-1-phenylethynyl-cyclohexanol
2-[4-(2-Chlor-phenyl)-piperazin-1-ylmethyl]-1-thiophen-2-yl-cyclohexanol
2-[4-(2-Chlor-phenyl)-piperazin-1-ylmethyl]-1-(3-methoxy-phenyl)-cyclohexanol
2-[4-(2-Chlor-phenyl)-piperazin-1-ylmethyl]-1-(3-phenyl-propyl)-cyclohexanol
2-[4-(2-Chlor-phenyl)-piperazin-1-ylmethyl]-1-p-tolyl-cyclohexanol
2-[4-(2-Chlor-phenyl)-piperazin-1-ylmethyl]-1-(4-methoxy-phenyl)-cyclohexanol
2-[4-(2-Chlor-phenyl)-piperazin-1-ylmethyl]-1-cyclohexylmethyl-cyclohexanol
2-[4-(2-Chlor-phenyl)-piperazin-1-ylmethyl]-1-(5-fluor-2-methoxy-phenyl)-cyclohexanol
1-(3-Chlor-phenyl)-2-[4-(2-chlor-phenyl)-piperazin-1-ylmethyl]-cyclohexanol
2-[4-(2-Chlor-phenyl)-piperazin-1-ylmethyl]-1-(3,5-dichlor-phenyl)-cyclohexanol
1-(2-Chlor-benzyl)-2-[4-(2-chlor-phenyl)-piperazin-1-ylmethyl]-cyclohexanol
2-[4-(2-Chlor-phenyl)-piperazin-1-ylmethyl]-1-(4-fluor-benzyl)-cyclohexanol
2-[4-(2-Chlor-phenyl)-piperazin-1-ylmethyl]-1-(3-methoxy-benzyl)-cyclohexanol
2-[4-(2-Chlor-phenyl)-piperazin-1-ylmethyl]-1-(4-chlor-3-trifluormethyl-phenyl)-cyclohexanol
2-[4-(2-Chlor-phenyl)-piperazin-1-ylmethyl]-1-(3-fluor-benzyl)-cyclohexanol
2-[4-(2-Chlor-phenyl)-piperazin-1-ylmethyl]-1-(2-methoxy-phenyl)-cyclohexanol
2-[4-(2-Chlor-phenyl)-piperazin-1-ylmethyl]-1-(2-methyl-benzyl)-cyclohexanol
1-(3-Chlor-4-fluor-phenyl)-2-[4-(2-chlor-phenyl)-piperazin-1-ylmethyl]-cyclohexanol
2-[4-(2-Chlor-phenyl)-piperazin-1-ylmethyl]-1-(3-trifluormethyl-phenyl)-cyclohexanol
2-[4-(2-Chlor-phenyl)-piperazin-1-ylmethyl]-1-(3-methyl-benzyl)-cyclohexanol
1-(4-Chlor-benzyl)-2-[4-(2-chlor-phenyl)-piperazin-1-ylmethyl]-cyclohexanol
1-(2-Chlor-6-fluor-benzyl)-2-[4-(2-chlor-phenyl)-piperazin-1-ylmethyl]-cyclohexanol
2-[4-(2-Chlor-phenyl)-piperazin-1-ylmethyl]-1-(2,5-dimethyl-benzyl)-cyclohexanol
1-(3-Chlor-benzyl)-2-[4-(2-chlor-phenyl)-piperazin-1-ylmethyl]-cyclohexanol
2-[4-(2-Chlor-phenyl)-piperazin-1-ylmethyl]-1-(2,4-dichlor-benzyl)-cyclohexanol
2-[4-(2-Methoxy-phenyl)-piperazin-1-ylmethyl]-1-phenyl-cyclohexanol
1-(4-Chlor-phenyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclohexanol
1-(4-Fluor-3-methyl-phenyl)-2-[4-(2-methoxy-phenyl)-piperazin-1-ylmethyl]-cyclohexanol
2-[4-(2-Methoxy-phenyl)-piperazin-1-ylmethyl]-1-o-tolyl-cyclohexanol
2-[4-(2-Methoxy-phenyl)-piperazin-1-ylmethyl]-1-vinyl-cyclohexanol
1-(4-tert-Butyl-phenyl)-2-[4-(2-methoxy-phenyl)-piperazin-1-ylmethyl]-cyclohexanol
1-Cyclopentyl-2-[4-(2-methoxy-phenyl)-piperazin-1-ylmethyl]-cyclohexanol
2-[4-(2-Methoxy-phenyl)-piperazin-1-ylmethyl]-1-m-tolyl-cyclohexanol
2-[4-(2-Methoxy-phenyl)-piperazin-1-ylmethyl]-bicyclohexyl-1-ol
1-(4-Fluor-phenyl)-2-[4-(2-methoxy-phenyl)-piperazin-1-ylmethyl]-cyclohexanol
2-[4-(2-Methoxy-phenyl)-piperazin-1-ylmethyl]-1-phenethyl-cyclohexanol
2-[4-(2-Methoxy-phenyl)-piperazin-1-ylmethyl]-1-phenylethynyl-cyclohexanol
2-[4-(2-Methoxy-phenyl)-piperazin-1-ylmethyl]-1-thiophen-2-yl-cyclohexanol
1-(3-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(3-phenyl-propyl)-cyclohexanol
1-(2,3-Dichlor-phenyl)-2-[4-(2-methoxy-phenyl)-piperazin-1-ylmethyl]-cyclohexanol
2-[4-(2-Methoxy-phenyl)-piperazin-1-ylmethyl]-1-p-tolyl-cyclohexanol
1-(4-Methoxy-phenyl)-2-[4-(2-methoxy-phenyl)-piperazin-1-ylmethyl]-cyclohexanol
1-Cyclohexylmethyl-2-[4-(2-methoxy-phenyl)-piperazin-1-ylmethyl]-cyclohexanol
1-(5-Fluor-2-methoxy-phenyl)-2-[4-(2-methoxy-phenyl)-piperazin-1-ylmethyl]-cyclohexanol
1-(3-Fluor-phenyl)-2-[4-(2-methoxy-phenyl)-piperazin-1-ylmethyl]-cyclohexanol
1-(3-Chlor-phenyl)-2-[4-(2-methoxy-phenyl)-piperazin-1-ylmethyl]-cyclohexanol
1-(3,5-Dichlor-phenyl)-2-[4-(2-methoxy-phenyl)-piperazin-1-ylmethyl]-cyclohexanol
1-(2-Chlor-benzyl)-2-[4-(2-methoxy-phenyl)-piperazin-1-ylmethyl]-cyclohexanol
1-(4-Fluor-benzyl)-2-[4-(2-methoxy-phenyl)-piperazin-1-ylmethyl]-cyclohexanol
1-(3-Methoxy-benzyl)-2-[4-(2-methoxy-phenyl)-piperazin-1-ylmethyl]-cyclohexanol
1-(4-Chlor-3-trifluormethyl-phenyl)-2-[4-(2-methoxy-phenyl)-piperazin-1-ylmethyl]-cyclohexanol
1-(3-Fluor-benzyl)-2-[4-(2-methoxy-phenyl)-piperazin-1-ylmethyl]-cyclohexanol
1-(2-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(2-methyl-benzyl)-cyclohexanol
1-(3-Chlor-4-fluor-phenyl)-2-[4-(2-methoxy-phenyl)-piperazin-1-ylmethyl]-cyclohexanol
2-[4-(2-Methoxy-phenyl)-piperazin-1-ylmethyl]-1-(3-trifluormethyl-phenyl)-cyclohexanol
2-[4-(2-Methoxy-phenyl)-piperazin-1-ylmethyl]-1-(3-methyl-benzyl)-cyclohexanol
1-(4-Chlor-benzyl)-2-[4-(2-methoxy-phenyl)-piperazin-1-ylmethyl]-cyclohexanol
1-(2-Chlor-6-fluor-benzyl)-2-[4-(2-methoxy-phenyl)-piperazin-1-ylmethyl]-cyclohexanol
1-(2,5-Dimethyl-benzyl)-2-[4-(2-methoxy-phenyl)-piperazin-1-ylmethyl]-cyclohexanol
1-(3-Chlor-benzyl)-2-[4-(2-methoxy-phenyl)-piperazin-1-ylmethyl]-cyclohexanol
1-(2,4-Dichlor-benzyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclododecanol
2-[4-(2-Methoxy-phenyl)-piperazin-1-ylmethyl]-1-vinyl-cyclododecanol
1-Phenethyl-2-[phenyl-(4-phenyl-piperazin-1-yl)-methyl]-cyclohexanol
1-Benzyl-2-[(4-methyl-piperazin-1-ylrphenyl-methyl]-cyclohexanol
1-Benzyl-2-[phenyl-(4-phenyl-piperazin-1-yl)-methyl]-cyclohexanol
2-[(4-Methyl-piperazin-1-yl)-phenyl-methyl]-1-phenethyl-cyclohexanol
2-[4-(2-Methoxy-phenyl)-piperazin-1-ylmethyl]-1-phenethyl-cyclododecanol
in Form des Diastereomeren, des Enantiomeren und eines Gemisches davon - einschließlich des Racemats - sowie in Form der entsprechenden Base, des entsprechenden Salzes und des entsprechenden Solvats.

Die Verwendung wenigstens einer Verbindung der allgemeinen Formel I, vorzugsweise der allgemeinen Formel II, III, IV, V, VI oder VII, zur Herstellung eines Arzneimittels zur Behandlung von neuropathischem, chronischem und/oder akutem Schmerz, zur Behandlung insbesondere mit neuropathischem Schmerz verbundener Symptome, zur Behandlung von Migräne, Hyperalgesie und/oder Allodynie, insbesondere thermischer Hyperalgesie, mechanischer Hyperalgesie, mechanischer Allodynie und/oder Kälte-Allodynie, zur Behandlung von inflammatorischem und/oder postoperativem Schmerz, zur Behandlung von neurodegenerativen Erkrankungen, wie Morbus Alzheimer, Morbus Huntington, Morbus Parkinson und/oder Epilepsie, zur Behandlung von psychatrischen und/oder neuropathologischen Störungen, wie bipolaren Störungen, Anxiety, Panikanfällen, Stimmungsschwankungen, manischem Verhalten, manisch-depressivem Verhalten, zur Behandlung von Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus, schmerzvoller diabetischer Neuropathie, zur Behandlung von Symptomen und/oder Schmerzen aufgrund von Multipler Sklerose und/oder der Parkinsonschen Krankheit, zur Behandlung von gastrointestinaler Schädigung, erythromelalgischem und/oder postpoliomyelitischem Schmerz, trigeminaler und/oder postherpetischer Neuralgie ist Gegenstand der Erfindung.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, formuliert und als solche auch verabreicht werden.

Neben wenigstens einer Verbindung der allgemeinen Formel 1, vorzugsweise der allgemeinen Formel II, III, IV, V, VI oder VII, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Verbindungen der allgemeinen Formel 1, vorzugsweise der allgemeinen Formel 11, III, IV, V, VI oder VII, in einem Depot in gelöster Form oder in einem Pflaster, gegebenenfalls unter Zusatz von die Hautpenetration fördernden Mitteln, sind geeignete perkutane Applikationszubereitungen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind.

Die an den Patienten zu verabreichende Menge der jeweiligen Verbindung der allgemeinen Formel I, vorzugsweise der allgemeinen Formel II, III, IV, V, VI oder VII, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer Verbindung der allgemeinen Formel I, vorzugsweise der allgemeinen Formel II, III, IV, V, VI oder VII, appliziert.

### Beispiele

### Pharmakologische Untersuchungen

Beim Bindungsassay wurde die Bindung und Affinitäten der erfindungsgemäßen Verbindungen zu der - auch in der Wissenschaft bisher noch unbekannten - Bindungsstelle von Gabapentin überprüft. Die Affinität der Verbindungen wurde über die Verdrängung von Gabapentin von seiner Bindungsstelle gemessen.

Zur Messung wurde wie folgt verfahren:
Komogenataliquote wurden aufgetaut und mit Puffer 1 20-fach verdünnt, resuspendiert und anschließend bei 110000 x g für 20 Minuten zentrifugiert. Das Pellet wurde mit Puffer 2 (10 mM HEPES, 0.05% NaN₃, 1 Tablette Complete (Roche Diagnostics) pro 100 ml, 0.004 % Struktol, pH 7.4) resuspendiert und auf eine Proteinkonzentration von 0.6 mg/ml eingestellt.
225 µL Homogenat, 20 µl [³H]-Gabapentin (76 Ci/mmol) in Puffer 2 entsprechend einer Konzentration von 10 nM im Testansatz sowie 5 µl einer erfindungsgemäßen Verbindung (gelöst in 25% DMSO in Wasser) mit einer Konzentration von 10 µM im Testansatz wurden auf einem Schüttler für 40 Minuten bei Raumtemperatur in 96-Well-Mikrotiterplatten (Polypropylen, Costar) inkubiert und anschließend mit einem Harvester (Robotic Harvester 9600, Brandel) auf 96-Well-Unifilterplatten mit GF/B-Filtem (Whatman) unter Vakuum abgesaugt. Die Filterplatten wurden vor dem Filtierschritt für 40 Minuten in Puffer 3 (50 mM Tris, 0.5 % PEI, pH 7.4) vorgequollen. Die Filterplatten wurden nach dem Filtierschritt dreimal mit Waschpuffer (0.1 M NaCl mit 0.05 NaN₃) gewaschen, um nichtgebundenen Radioliganden zu entfernen, und anschließend im Trockenschrank bei 55°C getrocknet. Nach einer Abkühlphase von 15 Minuten wurden pro Well 35 µl Szintillator (Ultima Gold MV, Packard) zugegeben und anschließend wurde im β-Counter (Trilux Microbeta, Wallac) die Radioaktivität auf den Filtern bestimmt.

Die Verbindungen der allgemeinen Formel I, vorzugsweise der allgemeinen Formel II, III, IV, V, VI oder VII, zeigten eine gute Hemmung bzw. Verdrängung von Gabapentin in diesem Assay. Die untersuchten Verbindungen wiesen daher in diesem einem biochemischen Assay eine Affinität zur bislang unbekannten Gabapentin-Bindungsstelle auf.

Man kann erwarten, daß die erfindungsgemäßen Verbindungen dem Gabapentin vergleichbare pharmakologische Eigenschaften entfalten, z.B. als Agenz gegen Schmerz oder Epilepsie. Dies wurde durch ihre analgetische Wirkung bewiesen. So verdrängen die erfindungsgemäßen Verbindungen nicht nur Gabapentin von seiner Bindungsstelle, sondern wirken auch - wie Gabapentin - deutlich analgetisch.

| | Gabapentin [%]-Hemmung |
|---|---|
| 1-(2-Chlor-benzyl)-2-(4-methyl-piperazin-1-ytmethyl)-cyclooctanol | 61 |
| 1-(4-Fluor-benzyl)-2-(4-methyl-piperazin-1-ylmethyl)-cyclooctanol | 39 |
| 1-(3-Fluor-benzyl)-2-(4-methyl-piperazin-1-ylmethyl)-cyclooctanol | 42 |
| 1-(2-Chlor-6-fluor-benzyl)-2-(4-methyl-piperazin-1-ylmethyl)-cyclooctanol | 60 |
| 1-(2,4-Dichlor-benzyl)-2-(4-methyl-piperazin-1-ylmethyl)-cyclooctanol | 48 |
| 1-(2-Chlor-benzyl)-2-(4-methyl-piperazin-1-ylmethyl)-cycloheptanol | 74 |
| 1-(3-Fluor-benzyl)-2-(4-methyl-piperazin-1-ylmethyl)-cycloheptanol | 44 |
| 1-(2-Chlor-6-fluor-benzyl)-2-(4-methyl-piperazin-1-ylmethyl)-cycloheptanol | 82 |
| 1-(2,4-Dichlor-benzyl)-2-(4-methyl-piperazin-1-ylmethyl)-cycloheptanol | 48 |
| 1-Benzyl-2-(4-methyl-piperazin-1-ylmethyl)-cyclododecanol | 56 |
| 2-(4-Methyl-piperazin-1-ylmethyl)-1-vinyl-cyclododecanol | 49 |
| 1-(3-Fluor-benzyl)-2-(4-methyl-piperazin-1-ylmethyl)-cyclododecanol | 45 |

## Patentansprüche

1. Verwendung wenigstens einer N,N'-disubstituierten Piperazin-Verbindung der allgemeinen Formel 1, 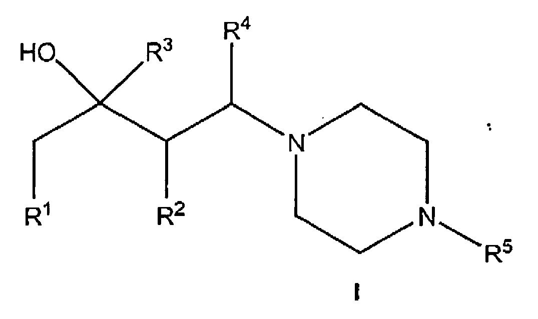 worin
R¹ und R², gleich oder verschieden, für jeweils einen linearen oder verzweigten, gesättigten oder ungesättigten aliphatischen Rest stehen oder zusammen eine (CH₂)ₙ-Kette bilden, wobei n für eine ganze Zahl steht,
R³ und R⁵, gleich oder verschieden, für jeweils einen linearen oder verzweigten, gesättigten oder ungesättigten aliphatischen Rest, einen gesättigten oder ungesättigten cycloaliphatischen Rest, einen Arylrest oder einen Heteroarylrest stehen, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann,
R⁴ für Wasserstoff oder einen gegebenenfalls einfach oder mehrfach substituierten Aryl- oder Heteroarylrest steht, wobei der Aryl- oder Heteroarylrest Teil eines polycyclischen Systems sein kann,
in Form ihrer Diastereomere, ihrer Enantiomere und Gemische davon einschließlich ihrer Racemate - sowie in Form entsprechender Basen, Salze und Solvate als Wirkstoff,
zur Herstellung eines Arzneimittels zur Behandlung von
• Epilepsie,
• neuropathischem, chronischem und/oder akutem Schmerz,
• mit neuropathischem Schmerz verbundener Symptome
• Migräne,
• Hyperalgesie, insbesondere thermischer Hyperalgesie und/oder mechanischer Hyperalgesie,
• Allodynie, insbesondere mechanischer Allodynie und/oder Kälte-Allodynie,
• inflammatorischem Schmerz,
• postoperativem Schmerz,
• neurodegenerativen Erkrankungen, insbesondere Morbus Alzheimer, Morbus Huntington und/oder Morbus Parkinson,
• psychiatrischen Störungen und/oder neuropathologischen Störungen, insbesondere bipolaren Störungen, Anxiety, Panikanfällen, Stimmungsschwankungen, manischem Verhalten und/oder manisch-depressivem Verhalten,
• Hitzewallungen,
• Beschwerden in der Postmenopause,
• Amyotropischer Lateraler Sklerose (ALS),
• Reflex Sympasthetic Dystrophy (RSD),
• Spastischer Lähmung,
• Restless Leg Syndrome,
• erworbenem Nystagmus,
• schmerzvoller diabetischer Neuropathie,
• Symptomen und/oder Schmerzen aufgrund von Multipler Sklerose und/oder der Parkinsonschen Krankheit,
• gastrointestinaler Schädigung,
• erythromelalgischem Schmerz,
• postpoliomyelitischem Schmerz,
• trigeminaler Neuralgie und/oder
• postherpetischer Neuralgie.

2. Verwendung gemäß Anspruch 1, **dadurch gekennzeichnet, daß**
R¹ und R², gleich oder verschieden, für jeweils einen linearen oder verzweigten, gesättigten oder ungesättigten aliphatischen C₁₋₆-Rest stehen oder zusammen eine (CH₂)ₙ-Kette bilden, wobei n für eine ganze Zahl von 2 - 9 steht,
R³ für einen linearen oder verzweigten, gesättigten oder ungesättigten aliphatischen C₁₋₆-Rest, einen gesättigten oder ungesättigten cycloaliphatischen C₃₋₇Rest, einen Phenylrest oder einen fünf oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann,
R⁴ für Wasserstoff oder einen Phenylrest steht,
R⁵ für einen linearen oder verzweigten, gesättigten oder ungesättigten aliphatischen C₁₋₆-Rest oder einen gegebenenfalls substituierten Phenylrest steht

3. Verwendung gemäß Anspruch 1, **dadurch gekennzeichnet, daß**
R¹ und R², gleich oder verschieden, für jeweils einen linearen oder verzweigten, gesättigten oder ungesättigten aliphatischen C₁₋₃-Rest stehen oder zusammen eine (CH₂)n-Kette bilden, wobei n für eine ganze Zahl von 2 - 9 steht,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁-₃-Brücke gebunden sein kann,
R⁴ für Wasserstoff oder einen Phenylrest steht,
R⁵ für einen linearen oder verzweigten, gesättigten oder ungesättigten aliphatischen C₁₋₃-Rest oder einen gegebenenfalls mit Halogen oder einer Alkoxygruppe substituierten Phenylrest steht.

4. Verwendung gemäß Anspruch 1, **dadurch gekennzeichnet, daß**
R¹ und R² jeweils für eine Methylgruppe stehen oder zusammen eine (CH₂)ₙ-Kette bilden, wobei n für 3, 4, 5 oder 9 steht,
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann,
R⁴ für Wasserstoff oder einen Phenylrest steht,
R⁵ für eine Methylgruppe oder einen gegebenenfalls mit Chlor oder einer Methoxygruppe substituierten Phenylrest steht.

5. Verwendung gemäß Anspruch 1 entsprechend der allgemeinen Formel II

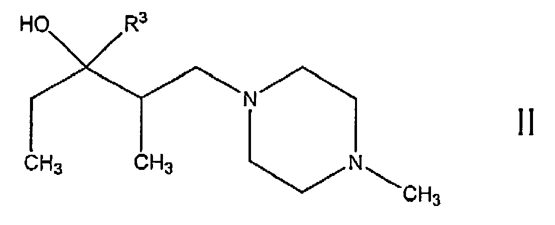

6. Verwendung gemäß Anspruch 1 entsprechend der allgemeinen Formel III, 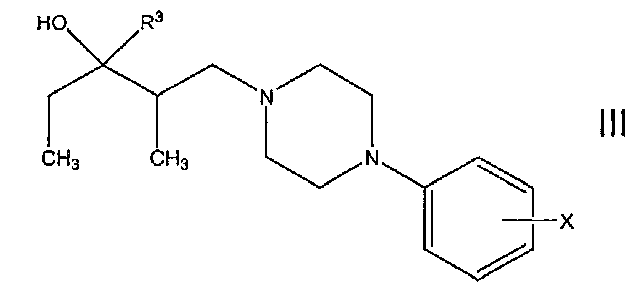 worin
X für Wasserstoff, Halogen oder eine Alkoxygruppe, vorzugsweise für Wasserstoff, Chlor oder eine Methoxygruppe, steht.

7. Verwendung gemäß Anspruch 1 entsprechend der allgemeinen Formel IV, 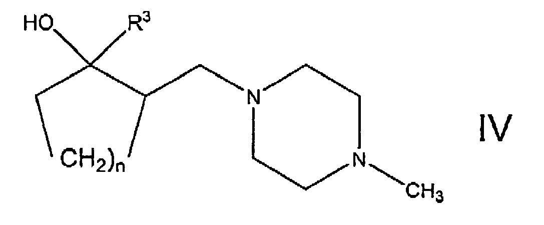 worin
n für eine ganze Zahl von 2-9 steht

8. Verwendung gemäß Anspruch 1 entsprechend der allgemeinen Formel V, 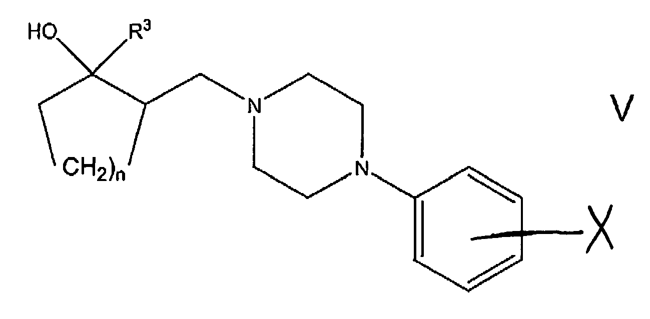 worin
n für eine ganze Zahl von 2 - 9 steht,
X für Wasserstoff, Halogen oder eine Alkoxygruppe, vorzugsweise für Wasserstoff, Chlor oder eine Methoxygruppe, steht.

9. Verwendung gemäß Anspruch 1 entsprechend der allgemeinen Formel VI, 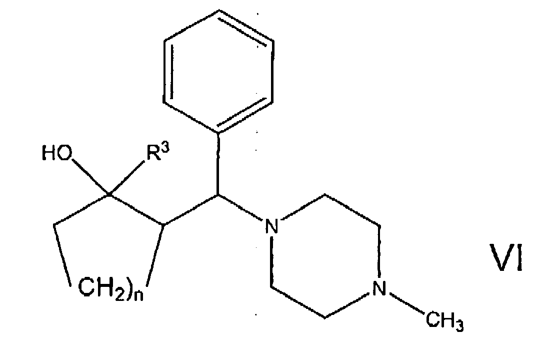 worin
n für eine ganze Zahl von 2 - 9 steht.

10. Verwendung gemäß Anspruch 1 entsprechend der allgemeinen Formel VII, 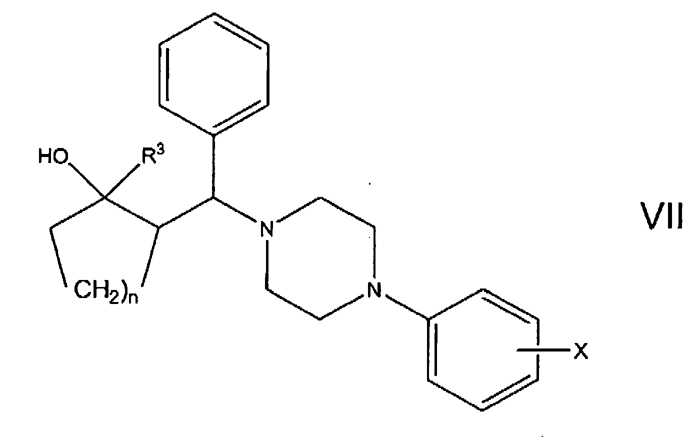 worin
n für eine ganze Zahl von 2 - 9 steht,
X für Wasserstoff, Halogen oder eine Alkoxygruppe, vorzugsweise für Wasserstoff, Chlor oder eine Methoxygruppe, steht.

11. Verwendung gemäß einem der Ansprüche 5-10, **dadurch gekennzeichnet, daß**
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann.

12. Verwendung gemäß einem der Ansprüche 5-10, **dadurch gekennzeichnet, daß**
R³ für einen linearen oder verzweigten, gesättigten oder ungesättigten aliphatischen C₁₋₃-Rest, einen gesättigten oder ungesättigten cycloaliphabschen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁₋₃-Brücke gebunden sein kann.

13. Verwendung gemäß einem der Ansprüche 5-10, **dadurch gekennzeichnet, daß**
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann.

14. Verwendung gemäß Anspruch 1, wobei die N,N'-disubstituierten Piperazin-Verbindung ausgewählt ist aus der Gruppe umfassend
2-Methyl-1-(4-methyl-piperazin-1-yl)-3-phenyl-pentan-3-ol
3-(4-Chlor-phenyl)-2-methyl-1-(4-methyl-piperazin-1-yl)-pentan-3-ol
3-Benzyl-2-methyl-1-(4-methyl-piperazin-1-yl)-pentan-3-ol
3-(4-Fluor-3-methyl-phenyl)-2-methyl-1-(4-methyl-piperazin-1-yl)-pentan-3-ol
2-Methyl-1-(4-methyl-piperazin-1-yl)-3-o-tolyl-pentan-3-ol
3-Ethyl-4-methyl-5-(4-methyl-piperazin-1-yl)-pent-1-en-3-ol
3-(4-tert-Butyl-phenyl)-2-methyl-1-(4-methyl-piperazin-1-yl)-pentan-3-ol
3-Cyclopentyl-2-methyl-1-(4-methyl-piperazin-1-yl)-pentan-3-ol
2-Methyl-1-(4-methyl-piperazin-1-yl)-3-m-tolyl-pentan-3-ol
3-Cyclohexyl-2-methyl-1-(4-methyl-piperazin-1-yl)-pentan-3-ol
3-(4-Fluor -phenyl)-2-methyl-1-(4-methyl-piperazin-1-yl)pentan-3-ol
3-Ethyl-2-methyl-1-(4-methyl-piperazin-1-yl)-5-phenyl-pentan-3-ol
3-Ethyl-4-methyl-5-(4-methyl-piperazin-1-yl)-1-phenyl-pent-1-yn-3-ol
2-Methy-1-(4-methyl-piperazin-1-yl)-3-thiophen-2-yl-pentan-3-ol
3-(3-Methoxy-phenyl)-2-methyl-1-(4-methyl-piperazin-1-yl)-pentan-3-ol
3-Ethyl-2-methyl-1-(4-methyl-piperazin-1-yl)-6-phenyl-hexan-3-ol
2-Methyl-1-(4-methyl-piperazin-1-yl)-3-p-tolyl-pentan-3-ol
3-(4-Methoxy-phenyl)-2-methyl-1-(4-methyl-piperazin-1-yl)-pentan-3-ol
2-(4-Methyl-piperazin-1-ylmethyl)-1-phenyl-cyclohexanol
1-Benzyl-2-(4-methyl-piperazin-1-ylmethyl)-cyclohexanol
1-(4-Fluor-3-methyl-phenyl)-2-(4-methyl-piperazin-1-ylmethyl)-cyclohexanol
2-(4-Methyl-piperazin-1-ylmethyl)-1-o-tolyl-cyclohexanol
2-(4-Methyl-piperazin-1-ylmethyl)-1-vinyl-cyclohexanol
1-Cyclopentyl-2-(4-methyl-piperazin-1-ylmethyl)-cyclohexanol
2-(4-Methyl-piperazin-1 -ylmethyl)-1-m-totyl-cyclohexanol
2-(4-Methyl-piperazin-1-ylmethyl)-bicyclohexyl-1-ol
1-(4-Fluor-phenyl)2-(4-methyl-piperazin-1-ylmethyl)-cyclohexanol
2-(4-Methyl-piperazin-1-ylmethyl) 1-phenethyl-cyclohexanol
2-(4-Methyl-piperazin-1-ylmethyl)-1-thiophen-2-yl-cyclohexanol
1-(2,4-Dichlor-phenyl)-2-(4-methl-piperazin-1-ylmethyl)-cyclohexanol
1-(3-Methoxy-phenyl)-2-(4-methyl-piperazin-1-ylmethyl)-cyclohexanol
2-(4-Methyl-piperazin-1-ylmethyl)-1-(3-phenyl-propyl)-cyclohexanol
2-(4-Methyl-piperazin-1 -ylmethyl)-1 -p-totyl-cyclohexanol
1-(4-Methoxy-phenyl)-2-(4-methyl-piperazin-1-ylmethyl)-cyclohexanol
1-Benzyl-2-(4-methyl-piperazin-1-ylmethyl)-cyclooctanol
2-(4-Methyl-piperazin-1-ylmethyl)-1-vinyl-cyclooctanol
1-(4-tert-Butyl-phenyl)-2-(4-methyl-piperazin-1-ylmethyl)cyclooctanol
1-Cyclopentyl-2-4-methyl-piperazin- ylmethyl)-cyclooctanol
2-(4-Methyl-piperazin-1-ylmethyl)-1-phenethyl-cyclooctanol
2-(4-Methyl-piperazin-1 -ylmethyl)-1-phenythynyl-cyclooctanol
2-(4-Methyl-piperazin-1-ylmethyl)-1-thiophen-2-yl-cyclooctanol
1-(3-Methoxy-phenyl)-2-(4-methyl-piperazin-1ylmethyl-cyclooctanol
2-(4-Methyl-piperazin-1-ylmethyl-1-phenyl-propyl)-cyclooctanol
2-(4-Methyl-piperazin-1-ylmethyl)-1-p-tolyl-cyclooctanol
1-(4-Methoxy-phenyl)-2-(4-methyl-piperazin-1-ylmethyl)cyclooctanol
2-(4-Methyl-piperazin-1-ylmethyl)-1-phenyl-cycloheptanol
1-(4-Chlor-phenyl)-2-(4-methyl-piperazin-1-ylmethyl)-cycloheptanol
1-Benzyl-2-(4-methyl-piperazin-1-ylmethyl)-cycloheptanol
1-(4-Fluor-3-methyl-phenyl)-2-(4-methyl-piperazin-1-ylmethyl)-cycloheptanol
2-(4-Methyl-piperazin-1-ylmethyl)-1-o-tolyl-cycloheptanol
2-(4-Methyl-piperazin-1-ylmethyl)-1-vinyl-cycloheptanol
1-(4-tert-Butyl-phenyl)-2-(4-methyl-piperazin-1-ylmethyl)-cycloheptanol
1-Cyclopentyl-2-(4-methyl-piperazin-1-ymethyl)-cycloheptanol
2-(4-Methyl-piperazin-1-ylmethyl)-1-m-tolyl-cycloheptanol
1-[4-(3-Chlor-phenyl)-piperazin-1-yl]-2-methyl-3-phenyl-pentan-3-ol
3-(4-Chlor-phenyl)-1-[4(3-chlor-phenyl)-piperazin-1-yl]-2-methyl-pentan-3-ol
3-Benzyl-1-[4(3-chlor-phenyl)-piperazin-1-yl]-2-methyl-pentan-3-ol
1-[4-(3-Chlor-phenyl)-piperazin-1-yl]-3-(4-fluor-3-methyl-phenyl)-2-methyl-pentan-3-ol
5-[4-(3-Chlor-phenyl)-piperazin-1-yl]-3-ethyl-4-methyl-pent-1-en-3-ol
3-(4-tert-Butyl-phenyl)-1-[4-(3-chlor-phenyl)-piperazin-1-yl]-2-methyl-pentan-3-ol
1-[4-(3-Chlor-phenyl)-piperazin-1-yl]-2-methyl-3-m-tolyl-pentan-3-ol
1-[4-(3-Chlor-phenyl)-piperazin-1-yl]-3-cyclohexyl-2-methyl-pentan-3-ol
1-[4-(3-Chlor-phenyl)-piperazin-1-yl]-3-(4-fluor-phenyl)-2-methyl-pentan-3-ol
1-[4-(3-Chlor-phenyl)-piperazin-1-yl]-3-ethyl-2-methyl-5-phenyl-pentan-3-ol
5-[4-(3-Chlor-phenyl)-piperazin-1-yl]-3-ethyl-4-methyl-1-phenyl-pent -1-yn-3-ol
1-[4-(3-Chlor-phenyl)-piperazin-1-yl]-2-methyl-3-thiophen-2-yl-pentan-3-ol
1-[4-(3-Chlor-phenyl)-piperazin-1-yl]-3-(3-methoxy-phenyl)-2-methyl-pentan-3-ol
1-[4-(3-Chlor-phenyl)-piperazin-1-yl]-ethyl-2-methyl-6-phenyl-hexan-ol
1-[4-(3-Chlor-phenyl)-piperazin-1-yl]-2-methyl-3-p-tolyl-pentan-3-ol
1-[4-(3-Chlor- phenyl)-piperazin-1-yl]-3-(4-methoxy-phenyl)-2-methyl-pentan-3-ol
1 -[4(3-Chlor-phenyl)-piperazin-1-yl]-3-(5-fluor-2-methoxy-phenyl)-2-methyl-pentan-3-ol
1-[4-(3-Chlor-phenyl)-piperazin-1-yl]-3-(3-fluor-phenyl)-2-methyl-pentan-3-ol
3-(3-Chlor-phenyl)-1-[4-(3-chlor-phenyl)-piperazin-yl]-2-methyl-pentan-3-ol
3-(2-Chlor-benzyl)-1-[4-(3-chlor-phenyl)-piperazin-1-yl]-2-methyl-pentan-3-ol
1-[4-(3-Chlor-phenyl)-piperazin-1-yl]-3-(4-fluor-benzyl)-2-methyl-pentan-3-ol
1-[4-(3-Chlor-phenyl)-piperazin-1-yl]-3-(3-methoxy-benzyl)-2-methyl-pentan-3-ol
1-[4-(3-Chlor-phenyl)-piperazin-1-yl]-3-(3-fluor-benzyl)-2-methyl-pentan-3-ol
1-[4-(3-Chlor-phenyl)-piperazin-1-yl]-3-(2-methoxy-phenyl)-2-methyl-pentan-3-ol
1-[4-(3-Chlor-phenyl)-piperazin-1-yl]-2-methyl-3-(2-methyl-benzyl)-pentan-3-ol 1-[4-(3-Chlor-phenyl)-piperazin-1-yl]-2-methyl-3-(3-trifluormethyl-phenyl)-pentan-3-ol
1-[4-(3-Chlor-phenyl)-piperazin-1-yl]-2-methyl-3-(3-methyl-benzyl)-pentan-3-ol
3-(4-Chlor-benzyl)-1-[4-(3(chlor-phenyl)-piperazin-1-yl]-2-methyl-pentan-3-ol
3-(2-Chlor-6-fluor-benzyl)-1-[4-(3-chlor-phenyl)-piperazin-1-yl]-2-methyl-pentan-3-ol
1-[4-(3-Chlor-phenyl)-piperazin-1 yl]-3-(2,5-dimethyl-benzyl)2-methyl-pentan-3-ol
(3-Chlor-benzyl)-1-[4-(3-chlor-phenyl)-piperazin-1-yl]-2-methyl-pentan-3-ol
1-[4-(3-Cchlor-phenyl)piperazin-1-yl]-3-(2,4-dichlor-benzyl)-2-methyl-pentan-3-ol
3-Cyclohexylmethyl-2-methyl-1-(4-methyl-piperazin-1-yl)-pentan-3-ol
3-(5-Fluor-2-methoxy-phenyl)-2-methyl-1-(4-methyl-piperazin-1-yl)-pentan-3-ol
3-(3-Fluor-phenyl)-2-methyl-1-(4-methyl-piperazin-1-yl)-pentan-3-ol
3-(3-Chlor-phenyl)-2-methyl-1-(4-methyl-piperazin-1-yl)-pentan-3-ol
3-(3,5-Dichlor-phenyl)2-methyl-1-(4-methyl-piperazin-1-yl)-pentan-3-ol
3-(2-Chlor-benzyl)-2-methyl-1-(4-methyl-piperazin-1-yl)-pentan-3-ol
3-(4-Fluor-benzyl)-2-methyl-1-(4-methyl-piperazin-1-yl)-pentan-3-ol
3-(3-Methoxy-benzyl)-2-methyl-1-(4-methyl-piperazin-1-yl)-pentan-3-ol
3-(4-Chlor-3-trifluormethyl-phenyl)-2-methyl-1-(4-methyl-piperazin-1-yl)-pentan-3-ol
3-(3-Fluor-benzyl)-2-methyl-1-(4-methyl-piperazin-1-yl)-pentan-3-ol
3-(2-Methoxy-phenyl)-2-methyl-1-(4-methyl-piperazin-1-yl)-pentan-3-ol
2-Methyl-3-(2-methyl-benzyl)-1-(4-methyl-piperazin-1-yl)-pentan-3-ol
3-(3-Chlor-4-fluor-phenyl)2-methyl-1-(4-methyl-piperazin-1-yl)-pentan-3-ol
2-Methyl-1-(4-methyl-piperazin-1-yl)-3-(3-trifluormethyl-phenyl)-pentan-3-ol
2-Methyl-3-(3-methyl-benzyl)-1 -(4-methyl-piperazin-1-yl)-pentan-3-ol
3-(4-Chlor-benzyl)-2-methyl-1-(4-methyl-piperazin-1 yl)-pentan-3-ol
3-(2-Chlor-6-fluor-benzyl)-2-methyl-1-(4-methyl-piperazin-1-yl)-pentan-3-ol
3-(2,5-Dimethyl-benzyl)-2-methyl-1-(4-methyl-piperazin-1-yl)-pentan-3-ol
3-(3-Chlor-benzyl)-2-methyl-1-(4-methyl-piperazin-1-yl)-pentan-3-ol
3-(2,4-Dichlor-benzyl)-2-methyl-1-(4methyl-piperazin-1-yl)-pentan-3-ol
1-Cyclohexylmethyl-2-(4-methyl-piperazin-1-ylmethyl)-cyclohexanol
1-(5-Fluor-2-methoxy-phenyl)-2-(4-methyl-piperazin-1 -ylmethyl)-cyclohexanol
1-(3-Fluor-phenyl)-2-(4-methyl-piperazin-1-ylmethyl)-cyclohexanol
1 -(3-Chlor-phenyl)-2-(4-methyl-piperazin-1-ylmethyl)-cyclohexanol
1-(3,5-Dichlor-phenyl)-2-(4-methyl-piperazin-1-ylmethyl)-cyclohexanol
1-(2-Chlor-benzyl)-2-(4-methyl-piperazin-1-ylmethyl)-cyclohexanol
1-(4-Fluor-benzyl)-2-(4-methyl-piperazin-1-ylmethyl)-cyclohexanol
1-(3-Methoxy-benzyl)-2-(4-methyl-piperazin-1-ylmethyl)-cyclohexanol
1-(4-Chlor-3-trifluormethyl-phenyl)-2-(4-methyl-piperazin-1-ylmethyl)-cyclohexanol
1-(3-Fluor-benzyl)-2-(4-methyl-piperazin-1-ylmethyl)-cyclohexanol
1-(2-Methoxy-phenyl)-2-(4-methyl-piperazin-1-ylmethyl)-cyclohexanol
1-(2-Methyl-benzyl)-2-(4-methyl-piperazin-1-ylmethyl)-cyclohexanol
1-(3-Cchlor-4-fluor-phenyl)-2-(4-methyl-piperazin-1-ylmethyl)cyclohexanol
2-(4-Methyl-piperazin-1-ylmetyl)-1-(3-trifluomethyl-phenyl)-cyclohexanol
1-(3-Methyl-benzyl)-2-(4-methyl-piperazin-1-ylmethyl)-cyclohexanol
1-(4-Chlor-benzyl)-2-(4-methyl-piperazin-1-ylmethyl)-cyclohexanol
1-(2,5-Dimethyl-benzyl)-2-(4-methyl-piperazin-1-ylmethyl)-cyclohexanol
1-(3-Chlor-benzyl)-2-(4-methyl-piperazin-1-ylmethyl)-cyclohexanol
1-(2,4-Dichlor-benzyl)-2-(4-methyl-piperazin-1-ylmethyl)-cyclohexanol
1-Cyclohexylmethyl-2-(4-methyl-piperazin-1-ylmethyl)-cyclooctanol
1-(5-Fluor-2-methoxy-phenyl)-2-(4-methyl-piperazin-1-ylmethyl)-Cyclooctanol
1-(2-Chlor-benzyl)-2-(4-methyl-piperazin-1-ylmethyl)-cyclooctanol
1-(4-Fluor-benzyl)-2-(4-methyl-piperazin-1-ylmethyl)-cyclootanol
1-(3-Fluor-benzyl)-2-(4-methyl-piperazin-1-ylmethyl)-cyclooctanol
1-(2-Methoxy-phenyl)-2-(4-methyl-piperazin-1-ylmethyl)-cyclooctanol
1-(3-Methyl-benzyl)-2-(4-methyl-piperazin-1-ylmethyl)-cyclooctanol
1-(4-Chlor-benzyl)-2-(4-methyl-piperazin-1-ylmethyl)-cyclooctanol
1-(2-Chlor-6-fluor-benzyl)-2-(4-methyl-piperazin-1-ylmethyl)-cyclooctanol
1-(2,5-Dimethyl-benzyl)-2-(4-methyl-piperazin-1-ylmethyl)-cyclooctanol
1-(3-Chlor-benzyl)-2-(4-methyl-piperazin-1-ylmethyl)-cyclooctanol
1-(2,4-Dichlor-benzyl)-2-(4-methyl-piperazin-1-ylmethyl)cyclooctanol
1-Cyclohexylmethyl-2-(4-methyl-piperazin-1-ylmethyl)-cycloheptanol
1-(5-Fluor-2-methoxy-phenyl)-2-(4-methyl-piperazin-1-ylmethyl)cycloheptanol
1-(3-Fluor-phenyl)-2-(4-methyl-piperazin-1-ylmethyl)-cycloheptanol
1-(3-Chlor-phenyl)-2-(4-methyl-piperazin-1-ylmethyl)-cycloheptanol
1-(3,5-Dichlor-phenyl)-2-(4-methyl-piperazin-1-ylmethyl)-cycloheptanol
1-(2-Chlor-benzyl)-2-(4-methyl-piperazin-1-ylmethyl)-cycloheptanol
1-(4-Fluor-benzyl)-2-(4-methyl-piperazin-1-ylmethyl)-cycloheptanol
1-(3-Methoxy-benzyl)-2-(4-methyl-piperazin-1-ylmethyl)-cycloheptanol
1-(4-Chlor-3-trifluormethyl-phenyl)-2-(4-methyl-piperazin-1-ylmethyl)-cycloheptanol
1-(3-Fluor-benzyl)-2-(4-methyl-piperazin-1-ylmethyl)-cycloheptanol
1-(2-Methoxy-phenyl)-2-(4-methyl-piperazin-1-ylmethyl)-cycloheptanol
1-(2-Methyl-benzyl)-2-(4-methyl-piperazin-1-ylmethyl)-cycloheptanol
1-(3-Chlo-4-fluor-phenyl)-2-(4-methyl-piperazin-1-ylmethyl)-cycloheptanol
2-(4-Methyl-piperazin-1-ylmethyl)-1-(3-trifluormethyl-phenyl)-cycloheptanol
1-(3-Methyl-benzyl)-2-(4-methyl-piperazin-ylmethyl)-cyocleptanol
1-(4-Chlor-benzyl)-2-(4-methyl-piperazin-1-ylmethyl)-cycloheptanol
1-(2-Cchlor-6-fluor-benzyl)-2-(4-methyl-piperazin-1-ylmethyl)-cycloheptanol
1-(2,5-Dimethyl-benzyl)-2-(4-methyl-piperazin-1-ylmethyl)-cycloheptanol
1-(3-Chlor-benzyl)-2-(4-methyl-piperazin-1-ylmethyl)-cycloheptanol
1-(2,4-Dichlor-benzyl)-2-(4-methyl-piperazin-1-ylmethyl)-cycloheptanol
1-Benzyl-2-(4-methyl-piperazin-1-ylmethyl)-cyclododecanol
2-(4-Methyl-piperazin-1-ylmethyl)-1-vinyl-cyclododecanol
1-Cyclopentyl-2-(4-methyl-piperazin-1-ylmethyl)-cyclododecanol
2-(4-Methyl-piperazin-1-ylmethyl)-1-phenethyl-cyclododecanol
2-(4-Methyl-piperazin-1-ylmethyl)-1-phenylethynyl-cyclododecanol
2-(4-Methyl-piperazin-1-ylmethyl)-1-thiophen-2-yl-cyclododecanol
2-(4-Methyl-piperazin-1-ylmethyl)-1-(3-phenyl-propyl)-cyclododecanol
1-(5-Fluor-2-methoxy-phenyl)-2-(4-methyl-piperazin-1-ylmethyl)cyclododecanol
1-(2-chlor-benzyl)-2-(4-methyl-piperazin-1-ylmethyl)-cyclododecanol
1-(4-Fluor-benzyl)-2-(4-methyl-piperazin-1-ylmethyl)-cyclododecanol
1-(3-Methoxy-benzyl)-2-(4-methyl-piperazin-1-ylmethyl)-cyclododecanol
1-(3-Fluor-benzyl)-2-(4-methyl-piperazin-1 -ylmethyl)-cyclododecanol
1-(2-Methoxy-phenyl)-2-(4-methyl-piperazin-1-ylmethyl)-cyclododecanol
1-(2-Methyl-benzyl-2-(4-methyl-piperazin-1-ylmethyl)cyclododecanol
1-(3-Methyl-benzyl)-2-(4-methyl-piperazin-1-ylmethyl)cyclododecanol
1-(4-Chlor-benzyl)-2-(4-methyl-piperazin-1-ylmethyl)-cyclododecanol
1-(2,5-Dimethyl-benzyl)-2-(4-methyl-piperazin-1-ylmethyl)-cyclododecanol
1-(3-chlor-benzyl)-2-(4-methyl-piperazin-1-ylmethyl)-cyclododecanol
1-(2,4-Dichlor-benzyl)-2-(4-methyl-piperazin-1-ylmethyl)-cyclododecanol
2-[4-(3-Chlor-phenyl)-piperazin-1-ylmethyl]-1-cyclohexylmethyl-cyclohexanol
2-[4-(3-Chlor-phenyl)-piperazin-1-ylmethyl]-1-(5-fluor-2-methoxy-phenyl)-cyclohexanol
2-[4-(3-Chlor-phenyl)-piperazin-1-ylmethy]-1-(3-fluor-phenyl)-cyclohexanol
1-(3-Chlor-phenyl)-2-[4-(3-chlor-phenyl)-piperazin-1-ylmethyl]cyclohexanol
2-[4-(3-Chlor-phenyl)-piperazin-1-ylmethyl]-1-(3,5-dichlor-phenyl)-cyclohexanol
1-(2-Chlor-benzyl)-2-[4-(3-chor-phenyl)-piperazin-1-ylmethyl]-cyclohexanol
2-[4-(3-Chlor-phenyl)-piperazin-1-ylmethyl]-1-(4-fluor-benzyl)-cyclohexanol
2-[4-(3-Chor-phenyl)-piperazin-1-ylmethyl]-1-(4-chlor-3-trifluomethylphenyl)-cydohexanol
2-[4-(3-Chlor-phenyl)-piperazin-1-ylmethyl]-1-(3-fluor-benzyl)-cyclohexanol
2-[4-(3-Chlor-phenyl)piperazin-1-ylmethyl]-1-(2-memoxy-phenyl)cyclohexanol
1-(3-Chlor-4-fluor-phenyl)-2-[4-(3-chlor-phenyl)-piperazin-1-ylmethyl]-cyclohexanol
2-[4-(3-Chlor-phenyl)-piperazin-1-ylmethyl]-1-(3-trifluormethyl-phenyl)cyclohexanol
2-[4-(3-Chlor-phenyl)-piperazin-1-ylmethyl]-1-(3-methyl-benzyl)-cyclohexanol
1-(4-Chlor-benzyl)-2-[4(3-chlor-phenyl)-piperazin-1-ylmethyl]cyclohexanol
1-(2-Chlor-6-fluor-benzyl)-2-[4-(3-chlor-phenyl)-piperazin-1-ylmethyl]-cyclohexanol
2-[4-(3-Chlor-phenyl)-piperazin-1-ylmethyl]-1-(2,5-dimethyl-benzyl)-cyclohexanol
1-(3-Chlor-benzyl)-2-[4{3-chlor-phenyl)-pipernzin-1-ylmethyl]-cyclohexanol
2-[4-(2-Chlor-phenyl)piperazin-1-ylmethyl]-1-phenyl-cyclohexanol
1-(4-Chlor-phenyl)-2-[4-(2-chlor-phenyl)-piperazin-1-ylmethyl]-cyclohexanol
1-Benzyl-2-[4-(2-chlor-phenyl)-piperazin-1-ylmethyl]-cyclohexanol
2-[4-(2-Chlor-phenyl)-piperazin-1-ylmethyl]-1-(4-fluor-3-methyl-phenyl)-cyclohexanol
2-[4-(2-Chlor-phenyl)piperazin-1-ylmethyl]-1-o-tolyl-cyclohexanol
2-[4-(2-Chlor-phenyl)-piperazin-1-ylmethyl]-1-vinyl-cyclohexanol
1-(4-tert-Butyl-phenyl)-2-[4-(2-chlor-phenyl)-piperazin-1-ylmethyl]-cyclohexanol
2-[4-(2-Chlor-phenyl)-piperazin-1-ylmethyl]-1-cyclopentyl-cyclohexanol
2-[4-(2-Chlor-phenyl)-piperazin-1-ylmethyl]-1-m-tolyl-cyclohexanol
2-[4(2-Chlor-phenyl)-piperazin-1-ylmethyl]-bicyclohexyl-1-ol
2-[4-(2-Chlor-phenyl)-piperazin-1-ylmethyl]-1-(4-fluor-phenyl)-cyclohexanol
2-[4-(2-chlor-phenyl)-piperazin-1-ylmethyl]-1-phenethyl-cydohexanol
2-[4-(2Chlor-phenyl)-piperazin-1-ylmethyl]-1-phenylethynyl-cyclohexanol
2-[4-(2-Chlor-phenyl)-piperazin-1-ylmethyl]-1-thiophen-2-yl-cyclohexanol
2-[4-(2-Chlor-phenyl)-piperazin-1-ylmethyl]-1-(3-methoxy-phenyl)-cyclohexanol
2-[4-(2-Chlor-phenyl)-piperazin-1-ylmethyl]-1-(3-phenyl-propyl)-cyclohexanol
2-[4-(2-Chlor-phenyl)piperazin-1-ylmethyl]-1-p-tolyl-cyclohexanol
2-[4-(2-Chlor-phenyl)-piperazin-1 yimethyl]-1-(4-methoxy-phenyl)-cyclohexanol
2-[4-(2-Chlor-phenyl)-piperazin-1-ylmethyl]-1-cyclohexylmethyl-cyclohexanol
2-[4-(2-Chlor-phenyl)-piperazin-1-ylmethyl]-1-(5-fluor-2-methoxy-phenyl)-cyclohexanol
1-(3-Chor-phenyl)-2-[4-(2-chlor-phenyl)-piperazin-1-ylmethyl]-cyclohexanol
2-[4-(2-chlor-phenyl)-piperazin-1-ylmethyl]-1-(3,5-dichlor-phenyl-cyclohexanol
1-(2-Chlor-benzyl)-2-[4-(2-chlor-phenyl)-piperazin-1-ylmethyl]-cyclohexanol
2-[4-(2-Chlor-phenyl)-piperezin-1-ylmethyl]-1-(4-fluor-benzyl)-cyclohexanol
2-[4-(2-Chlor-phenyl-piperazin-1-ylmethyl]-1-(3-methoxy-benzyl)cyclohexanol
2-[4-(2-Chlor-phenyl)-piperazin-1-ylmethyl]-1-(4-chlor-3-trifluormethyl-phenyl)-cyclohexanol
2-[4-(2-Chlor-phenyl)piperazin-1-ylmethyl]-1-(3-fluor-benzyl)-cyclohexanol
2-[4-(2-Chlor-phenyl)-piperazin-1-ylmethyl]-1-(2-methoxy-phenyl)-cyclohexanol
2-[4-(2-Chlor-phenyl)-piperazin-1-ylmethyl]-1-(2-methyl-benzyl)-cyclohexanol
1-(3-Chlor-4-fluor-phenyl)-2-[4-(2-chlor-phenyl)-piperazin-1-ylmethyl]-cyclohexanol
2-[4-(2-Chlor-phenyl)-piperazin-1-ylmethyl]-1-(3-trifluormethyl-phenyl)-cyclohexanol
2-[4-(2-Chlor-phenyl)-piperazin-1-ylmethyl]-1-(3-methyl-benzyl)-cyclohexanol
1-(4-Chlor-benzyl)-2-[4-(2-chlor-phenyl)-piperazin-1-ylmethyl]-cyclohexanol
1-(2-Chlor-6-fluor-benzyl)-2-[4-(2-chlor-phenyl)-piperazin-1-ylmethyl]-cyclohexanol
2-[4-(2-Chlor-phenyl)-piperazin-1-ylmethyl]-1-(2,5-dimethyl-benzyl)-cyclohexanol
1-(3-Chlor-benzyl)-2-[4-(2-chlor-phenyl)-piperazin-1-ylmethyl]-cyclohexanol
2-[4-(2-Chlor-phenyl)piperazin-1-ylmethyl]-1-(2,4-dichlor-benzyl)-cyclohexanol
2-[4-(2-methoxy-phenyl)-piperazin-1-ylmethyl]-1-phenyl-cyclohexanol
1-(4-chlor-phenyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclohexanol
1-(-4-Fluor-3-methyl-phenyl)-2-[4-(2-methoxy-phenyl)-piperazin-1-ylmethyl]-cydohexanol
2-[4-(2-Methoxy-phenyl)-piperazin-1-ylmethyl]-1-o-tolyl-cyclohexanol
2-[4-(2-Methoxy-phenyl)-piperazin-1-ylmethyl]-1-vinyl-cyclohexanol
1-(4-tert-Butyl-phenyl)2-[4-(2-methoxy-phenyl)piperazin-1-ylmethyl]-cyclohexanol
1-Cyclopentyl-2-[4-(2-methoxy-phenyl)-piperazin-1-ylmethyl]-cyclohexanol
2-[4-(2-Methoxy-phenyl)-piperazin-1-ylmethyl]-1-m-tolyl-cyclohexanol
2-[4-(2-Methoxy-phenyl)piperazin-1-ylmethyl]-bicyclohexyl-1-ol
1-(4-Fluor-phenyl)-2-[4-(2-methoxy-phenyl)-piperazin-1-ylmethyl]-cyclohexanol
2-[4-(2-Methoxy-phenyl)-piperazin-1-ylmethyl]-1-phenethyl-cyclohexanol
2-[4-(2-Methoxy-phenyl)-piperazin-1-ylmethyl]-1-phenylethynyl-cyclohexanol
2-[4-(2-Methoxy-phenyl)-piperazin-1 -ylmethyl]-1-thiophen-2-yl-cyclohexanol
1 -(3-Methoxy-phenyl)-2-[4-(2-methoxy-pheny)-piperazin-1-ylmethyl]-cyclohexanol
2-[4-(2-Methoxy-phenyl)piperazin-1-ylmethyl]-1-3-phenyl-propyl)-cyclohexanol
1 -(2,3-Dichlor-phenyl)-2-[4-(2-methoxy-phenyl)-piperazin-1 -ylmethyl]-cyclohexanol
2-[4-(2-Methoxy-phenyl)-piperazin-1-ylmethyl]-1-p-tolyl-cyclohexanol
1-(4-Methoxy-phenyl)2-[4-(2-methoxy-phenyl)-piperazin-1-ylmethyl]cyclohexanol
1-Cyclohexylmethyl-2-[4-(2-methoxy- phenyl)-piperazin-1-ylmethyl]-cyclohexanol
1-(5-Fluor-2-methoxy-phenyl)-2-[4-(2-methoxy-phenyl)-piperazin-1-ylmethyl]-cyclohexanol
1-(3-Fluor-phenyl)-2-[4-(2-methoxy-phenyl)-piperazin-1 ylmethyl]-cyclohexanol
1-(3-Chlor-phenyl)-2-[4-(2-methoxy-phenyl)-piperazin-1-ylmethyl]-cyclohexanol
1-(3,5-Dichlor-phenyl)-2-[4-(2-methoxy-phenyl)-piperazin-1-ylmethyl]-cyclohexanol
1-(2-Chlor-benzyl)-2-[4-(2-methoxy-phenyl)-piperizan-1-ylmethyl]-cyclohexanol
1-(4-Fluor-benzyl)-2-[4-(2-methoxy-phenyl)-piperazin-1-ylmethyl]-cyclohexanol
1-(3-Methoxy-benzyl)-2-[4-(2-methoxy-phenyl)-piperazin-1-ylmethyl]-cyclohexanol
1-(4-Chlor-3-trifluormethyl-phenyl)-2-[4(2-methoxy-phenyl)-piperizan-1-ylmethyl]-cyclohexanol
1-(3-Fluor-benzyl)-2-[4-(2-methoxy-phenyl)-piperazin-1-ylmethyl]-cyclohexanol
1-(2-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(2-methyl-benzyl)-cyclohexanol
1-(3-Chlor-4-fluor-phenyl)-2-[4-(2-methoxy-phenyl)-piperazin-1-ylmethyl]-cyclohexanol
2-[4-(2-Methoxy-phenyl)-piperazin-1 -ylmethl]-1 -(3-trifluomethyl-phenyl)-cyclohexanol
2-[4-(2-Methoxy-phenyl)-piperazin-1-ylmethyl]-1-(3-methyl-benzyl)-cyclohexanol
1-(4-Chlor-benzyl)-2-[4-(2-methoxy-phenyl)-piperazin-1-ylmethyl]-cyclohexanol
1-(2-Chlor-6-fluor-benzyl)-2-[4-(2-methoxy-phenyl)-piperazn-1- ylmethyl]-cyclohexanol
1-(2,5-Dimethyl-benzyl)-2-[4-(2methoxy-phenyl)-piperizan-1-ylmethyl]-Cyclohexanol
1-(3-Chlor-benzyl)-2-[4-(2-methoxy-phenyl-piperazin-1-ylmethyl]-cyclohexanol
1-(2,4-Dichlor-benzyl)-2-[4-(2-methoxy-phenyl)-piperazin-1-ylmethyl]-cyclohexanol
1-Benzyl-2-[4-(2-methoxy-phenyl)-piperazin-1-ylmethy]-cyclododecanol
2-[4-(2-Methoxy-phenyl)-piperazin-1-ylmethyl]-1-vinyl-cyclododecanol
1-Phenethyl-2-[phenyl-(4-phenyl-piperazin-1-yl)methyl]-cyclohexanol
1-Benzyl-2-[(4-methyl-piperazin-1-yl)-phenyl-methyl]-cyclohexanol
1-Benzyl-2-[phenyl-(4-phenyl-piperazin-1-yl)-methyl]-cyclohexanol
2-[(4-Methyl-piperzin-1-yl)-phenyl-methyl]-1-phenethyl-cyclohexanol and
2-[4-(2-Methoxy-phenyl)-piperazin-1-ylmethyl]-1-phenethyl-cyclododecanol
in Form des Diastereomeren, des Enantiomeren und eines Gemisches davon - einschließlich des Racemats - sowie in Form der entsprechenden Base, des entsprechenden Salzes und des entsprechenden Solvats.

## Claims

1. Use of at least one N,N'-disubstituted piperazine compound of the general formula I, 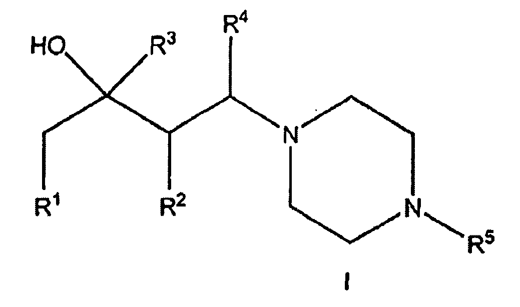 in which
R¹ and R², identical or different, in each case denote a linear or branched, saturated or unsaturated aliphatic residue, or together form a (CH₂)ₙ chain, wherein n denotes an integer,
_{R}³ and R⁵, identical or different, in each case denote a linear or branched, saturated or unsaturated aliphatic residue, a saturated or unsaturated cycloaliphatic residue, an aryl residue or a heteroaryl residue, wherein the respective ring system may be optionally mono- or polysubstituted and/or attached via a linear or branched, saturated or unsaturated aliphatic bridge and/or the aryl or heteroaryl residue may be part of a polycyclic system,
R⁴ denotes hydrogen or an optionally mono- or polysubstituted aryl or heteroaryl residue, wherein the aryl or heteroaryl residue may be part of a polycyclic system,
in the form of the diastereomers thereof, the enantiomers thereof and mixtures thereof - including the racemates thereof - and in the form of corresponding bases, salts and solvates as active ingredient,
for the production of a medicament for the treatment of
• epilepsy,
• neuropathic, chronic and/or acute pain,
• symptoms associated with neuropathic pain,
• migraine,
• hyperalgesia, in particular thermal hyperalgesia and/or mechanical hyperalgesia,
• allodynia, in particular mechanical allodynia and/or cold allodynia,
• inflammatory pain,
• postoperative pain,
• neurodegenerative diseases, in particular Alzheimer's disease, Huntington's chorea and/or Parkinson's disease,
• psychiatric disorders and/or neuropathological disorders, in particular bipolar disorders, anxiety, panic attacks, mood swings, manic behaviour and/or manicdepressive behaviour,
• hot flushes,
• postmenopausal complaints,
• amyotropic lateral sclerosis (ALS),
• reflex sympathetic dystrophy (RSD),
• spastic paralysis,
• restless leg syndrome,
• acquired nystagmus,
• painful diabetic neuropathy,
• symptoms and/or pain due to multiple sclerosis and/or Parkinson's disease,
• gastrointestinal damage,
• erythromelalgic pain,
• postpoliomyelitic pain,
• trigeminal neuralgia and/or
• posttherapeutic neuralgia.

2. Use according to claim 1, **characterised in that**
R¹ and R², identical or different, in each case denote a linear or branched, saturated or unsaturated aliphatic C₁₋₆ residue, or together form a (CH₂)ₙ chain, wherein n denotes an integer from 2-9,
R³ denotes a linear or branched, saturated or unsaturated aliphatic C₁₋₆ residue, a saturated or unsaturated cycloaliphatic C₃₋₇ residue, a phenyl residue or a five- or six-membered heteroaryl residue, wherein the respective ring system may optionally be mono- or polysubstituted and/or attached via a linear or branched, saturated or unsaturated aliphatic C₁₋₅ bridge,
R⁴ denotes hydrogen or a phenyl residue,
R⁵ denotes a linear or branched, saturated or unsaturated aliphatic C₁₋₅ residue or an optionally substituted phenyl residue.

3. Use according to claim 1, **characterised in that**
R¹ and R², identical or different, in each case denote a linear or branched, saturated or unsaturated aliphatic C₁₋₃ residue, or together form a (CH₂)ₙ chain, wherein n denotes an integer from 2-9,
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue, wherein the respective ring system may optionally be mono- or polysubstituted with halogen, an alkyl group, an alkoxy group and/or a trihalogenated alkyl group and/or may be attached via a linear or branched, saturated or unsaturated aliphatic C₁₋₃ bridge,
R⁴ denotes hydrogen or a phenyl residue,
R⁵ denotes a linear or branched, saturated or unsaturated aliphatic C₁₋₃ residue or a phenyl residue optionally substituted with halogen or an alkoxy group.

4. Use according to claim 1, **characterised in that**
R¹ and R² in each case denote a methyl group, or together form a (CH₂)ₙ chain, wherein n denotes 3, 4, 5 or 9,
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge,
R⁴ denotes hydrogen or a phenyl residue,
R⁵ denotes a methyl group or a phenyl residue optionally substituted with chlorine or a methoxy group.

5. Use according to claim 1 corresponding to the general formula II

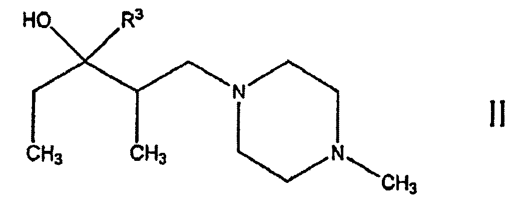

6. Use according to claim 1 corresponding to the general formula III, 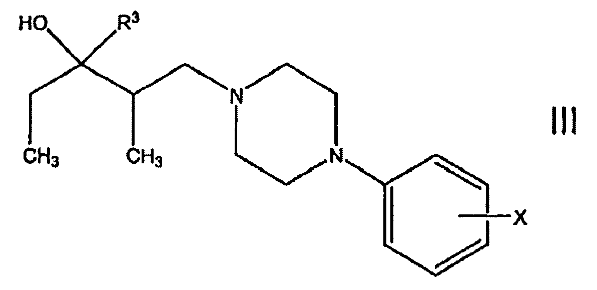 in which
X denotes hydrogen, halogen or an alkoxy group, preferably hydrogen, chlorine or a methoxy group.

7. Use according to claim 1 corresponding to the general formula IV, 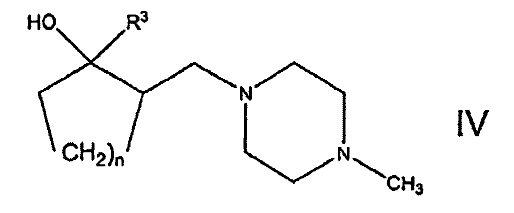 in which
n denotes an integer from 2-9.

8. Use according to claim 1 corresponding to the general formula V, 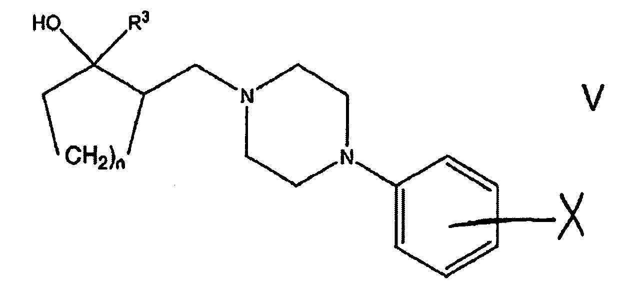 in which
n denotes an integer from 2-9,
X denotes hydrogen, halogen or an alkoxy group, preferably hydrogen, chlorine or a methoxy group.

9. Use according to claim 1 corresponding to the general formula VI, 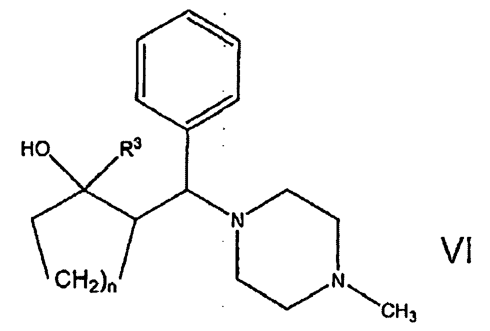 in which
n denotes an integer from 2-9.

10. Use according to claim 1 corresponding to the general formula VII, 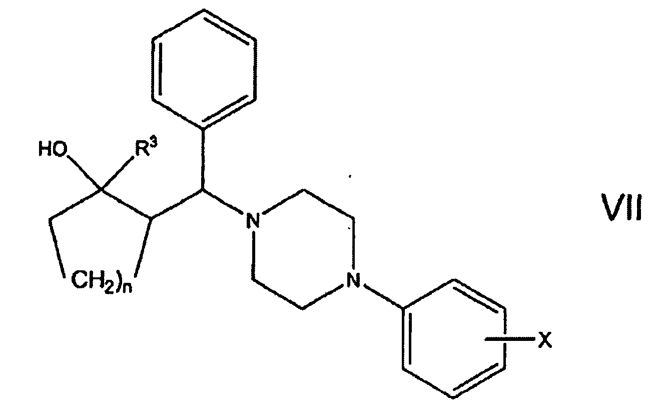 in which
n denotes an integer from 2-9,
X denotes hydrogen, halogen or an alkoxy group, preferably hydrogen, chlorine or a methoxy group.

11. Use according to any one of claims 5-10, **characterised in that**
R³ denotes a linear or branched, saturated or unsaturated aliphatic C₁₋₆ residue, a saturated or unsaturated cycloaliphatic C₃₋₇ residue, a phenyl residue or a five- or six-membered heteroaryl residue, wherein the respective ring system may optionally be mono- or polysubstituted and/or attached via a linear or branched, saturated or unsaturated aliphatic C₁₋₅ bridge.

12. Use according to any one of claims 5-10, **characterised in that**
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue, wherein the respective ring system may optionally be mono- or polysubstituted with halogen, an alkyl group, an alkoxy group and/or a trihalogenated alkyl group and/or may be attached via a linear or branched, saturated or unsaturated aliphatic C₁₋₃ bridge.

13. Use according to any one of claims 5-10, **characterised in that**
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge.

14. Use according to claim 1, wherein the N,N'-disubstituted piperazine compound is selected from the group comprising
2-methyl-1-(4-methylpiperazin-1-yl)-3-phenylpentan-3-ol
3-(4-chlorophenyl)-2-methyl-1-(4-methylpiperazin-1-yl)-pentan-3-ol
3-benzyl-2-methyl-1-(4-methylpiperazin-1-yl)-pentan-3-ol
3-(4-fluoro-3-methylphenyl)-2-methyl-1-(4-methylpiperazin-1-yl)-pentan-3-ol
2-methyl-1-(4-methylpiperazin-1-yl)-3-o-tolylpentan-3-ol
3-ethyl-4-methyl-5-(4-methylpiperazin-1-yl)-pent-1-en-3-ol
3-(4-tert-butylphenyl)-2-methyl-1-(4-methylpiperazin-1-yl)-pentan-3-ol
3-cyclopentyl-2-methyl-l-(4-methylpiperazin-1-yl)-pentan-3-ol
2-methyl-1-(4-methylpiperazin-1-yl)-3-m-tolylpentan-3-ol
3-cyclohexyl-2-methyl-1-(4-methylpiperazin-1-yl)-pentan-3-ol
3-(4-fluorophenyl)-2-methyl-1-(4-methylpiperazin-1-yl)-pentan-3-ol
3-ethyl-2-methyl-1-(4-methylpiperazin-1-yl)-5-phenylpentan-3-ol
3-ethyl-4-methyl-5-(4-methylpiperazin-1-yl)-1-phenylpent-1-yn-3-ol
2-methyl-1-(4-methylpiperazin-1-yl)-3-thiophen-2-ylpentan-3-ol
3-(3-methoxyphenyl)-2-methyl-1-(4-methylpiperazin-1-yl)-pentan-3-ol
3-ethyl-2-methyl-(4-methylpiperazin-1-yl)-6-phenylhexan-3-ol
2-methyl-1-(4-methylpiperazin-1-yl)-3-p-tolylpentan-3-ol
3-(4-methoxyphenyl)-2-methyl-1-(4-methylpiperazin-1-yl)-pentan-3-ol
2-(4-methylpiperazin-1-ylmethyl)-1-phenyl-cyclohexanol
1-benzyl-2-(4-methylpiperazin-1-ylmethyl)-cyclohexanol
1-(4-fluoro-3-methylphenyl)-2-(4-methylpiperazin-1-ylmethyl)-cyclohexanol
2-(4-methylpiperazin-1-ylmethyl)-1-o-tolyl-cyclohexanol
2-(4-methylpiperazin-1-ylmethyl)-1-vinyl-cyclohexanol
1-cyclopentyl-2-(4-methylpiperazin-1-ylmethyl)-cyclohexanol
2-(4-methylpiperazin-1-ylmethyl)-1-m-tolyl-cyclohexanol
2-(4-methylpiperazin-1-ylmethyl)-bicyclohexyl-1-ol
1-(4-fluorophenyl)-2-(4-methylpiperazin-1-ylmethyl)-cyclohexanol
2-(4-methylpiperazin-1-ylmethyl)-1-phenethyl-cyclohexanol
2-(4-methylpiperazin-1-ylmethyl)-1-thiophen-2-yl-cyclohexanol
1-(2,4-dichlorophenyl)-2-(4-methylpiperazin-1-ylmethyl)-cyclohexanol
1-(3-methoxyphenyl)-2-(4-methylpiperazin-1-ylmethyl)-cyclohexanol
2-(4-methylpiperazin-1-ylmethyl)-1-(3-phenylpropyl)-cyclohexanol
2-(4-methylpiperazin-1-ylmethyl)-1-p-tolyl-cyclohexanol
1-(4-methoxyphenyl)-2-(4-methylpiperazin-1-ylmethyl)-cyclohexanol
1-benzyl-2-(4-methylpiperazin-1-ylmethyl)-cyclooctanol
2-(4-methylpiperazin-1-ylmethyl)-1-vinyl-cyclooctanol
1-(4-tert-butylphenyl)-2-(4-methylpiperazin-1-ylmethyl)-cyclooctanol
1-cyclopentyl-2-(4-methylpiperazin-1-ylmethyl)-cyclooctanol
2-(4-methylpiperazin-1-ylmethyl)-1-phenethyl-cyclooctanol
2-(4-methylpiperazin-1-ylmethyl)-1-phenylethynylcyclooctanol
2-(4-methylpiperazin-1-ylmethyl)-1-thiophen-2-ylcyclooctanol
1-(3-methoxyphenyl)-2-(4-methylpiperazin-1-ylmethyl)-cyclooctanol
2-(4-methylpiperazin-1-ylmethyl)-1-(3-phenylpropyl)-cyclooctanol
2-(4-methylpiperazin-1-ylmethyl)-1-p-tolylcyclooctanol
1-(4-methoxyphenyl)-2-(4-methylpiperazin-1-ylmethyl)-cyclooctanol
2-(4-methylpiperazin-1-ylmethyl)-1-phenylcycloheptanol
1-(4-chlorophenyl)-2-(4-methylpiperazin-1-ylmethyl)-cycloheptanol
1-benzyl-2-(4-methylpiperazin-1-ylmethyl)-cycloheptanol
1-(4-fluoro-3-methylphenyl)-2-(4-methylpiperazin-1-ylmethyl)-cycloheptanol
2-(4-methylpiperazin-1-ylmethyl)-1-o-tolylcycloheptanol
2-(4-methylpiperazin-1-ylmethyl)-1-vinyl-cycloheptanol
1-(4-tert-butylphenyl)-2-(4-methylpiperazin-1-ylmethyl)-cycloheptanol
1-cyclopentyl-2-(4-methylpiperazin-1-ylmethyl)-cycloheptanol
2-(4-methylpiperazin-1-ylmethyl)-1-m-tolylcycloheptanol
1-[4-(3-chlorophenyl)-piperazin-1-yl]-2-methyl-3-phenylpentan-3-ol
3-(4-chlorophenyl)-1-[4-(3-chlorophenyl)-piperazin-1-yl]-2-methylpentan-3-ol
3-benzyl-1- [4-(3-chlorophenyl)-piperazin-1-yl]-2-methylpentan-3-ol
1-[4-(3-chlorophenyl)-piperazin-1-yl]-3-(4-fluoro-3-methylphenyl)-2-methylpentan-3-ol
5-[4-(3-chlorophenyl)-piperazin-1-yl]-3-ethyl-4-methylpent-1-en-3-ol
3-(4-tert-butylphenyl)-1-[4-(3-chlorophenyl)-piperazin-1-yl]-2-methylpentan-3-ol
1-[4-(3-chlorophenyl)-piperazin-1-yl]-2-methyl-3-m-tolylpentan-3-ol
1-[4-(3-chlorophenyl)-piperazin-1-yl]-3-cyclohexyl-2-methylpentan-3-ol
1-[4-(3-chlorophenyl)-piperazin-1-yl]-3-(4-fluorophenyl)-2-methylpentan-3-ol
1-[4-(3-chlorophenyl)-piperazin-1-yl]-3-ethyl-2-methyl-5-phenylpentan-3-ol
5-[4-(3-chlorophenyl)-piperazin-1-yl]-3-ethyl-4-methyl-1-phenylpent-1-yn-3-ol
1-[4-(3-chlorophenyl)-piperazin-1-yl]-2-methyl-3-thiophen-2-ylpentan-3-ol
1-[4-(3-chlorophenyl)-piperazin-1-yl]-3-(3-methoxyphenyl)-2-methylpentan-3-ol
1-[4-(3-chlorophenyl)-piperazin-1-yl]-3-ethyl-2-methyl-6-phenyl-hexan-3-ol
1-[4-(3-chlorophenyl)-piperazin-1-yl]-2-methyl-3-p-tolylpentan-3-ol
1-[4-(3-chlorophenyl)-piperazin-1-yl]-3-(4-methoxyphenyl)-2-methylpentan-3-ol
1-[4-(3-chlorophenyl)-piperazin-1-yl]-3-(5-fluoro-2-methoxyphenyl)-2-methylpentan-3-ol
1-[4-(3-chlorophenyl)-piperazin-1-yl]-3-(3-fluorophenyl)-2-methylpentan-3-ol
3-(3-chlorophenyl)-1-[4-(3-chlorophenyl)-piperazin-1-yl]-2-methylpentan-3-ol
3-(2-chlorobenzyl)-1-[4-(3-chlorophenyl)-piperazin-1-yl]-2-methylpentan-3-ol
1-[4-(3-chlorophenyl)-piperazin-1-yl]-3-(4-fluorobenzyl)-2-methylpentan-3-ol
1-[4-(3-chlorophenyl)-piperazin-1-yl]-3-(3-methoxybenzyl)-2-methylpentan-3-ol
1-[4-(3-chlorophenyl)-piperazin-1-yl]-3-(3-fluorobenzyl)-2-methylpentan-3-ol
1-[4-(3-chlorophenyl)-piperazin-1-yl]-3-(2-methoxyphenyl)-2-methylpentan-3-ol
1-[4-(3-chlorophenyl)-piperazin-1-yl]-2-methyl-3-(2-methylbenzyl)-pentan-3-ol
1-[4-(3-chlorophenyl)-piperazin-1-yl]-2-methyl-3-(3-trifluoromethylphenyl)-pentan-3-ol
1-[4-(3-chlorophenyl)-piperazin-1-yl]-2-methyl-3-(3-methylbenzyl)-pentan-3-ol
3-(4-chlorobenzyl)-1-[4-(3-chlorophenyl)-piperazin-1-yl]-2-methylpentan-3-ol
3-(2-chloro-6-fluorobenzyl)-1-[4-(3-chlorophenyl)-piperazin-1-yl]-2-methylpentan-3-ol
1-[4-(3-chlorophenyl)-piperazin-1-yl]-3-(2,5-dimethylbenzyl)-2-methylpentan-3-ol
3-(3-chlorobenzyl)-1-[4-(3-chlorophenyl)-piperazin-1-yl]-2-methylpentan-3-ol
1-[4-(3-chlorophenyl)-piperazin-1-yl]-3-(2,4-dichlorobenzyl)-2-methylpentan-3-ol
3-cyclohexylmethyl-2-methyl-1-(4-methylpiperazin-1-yl)-pentan-3-ol
3-(5-fluoro-2-methoxyphenyl)-2-methyl-1-(4-methylpiperazin-1-yl)-pentan-3-ol
3-(3-fluorophenyl)-2-methyl-1-(4-methylpiperazin-1-yl)-pentan-3-ol
3-(3-chlorophenyl)-2-methyl-1-(4-methylpiperazin-1-yl)-pentan-3-ol
3-(3,5-dichlorophenyl)-2-methyl-1-(4-methylpiperazin-1-yl)-pentan-3-ol
3-(2-chlorobenzyl)-2-methyl-1-(4-methylpiperazin-1-yl)-pentan-3-ol
3-(4-fluorobenzyl)-2-methyl-1-(4-methylpiperazin-1-yl)-pentan-3-ol
3-(3-methoxybenzyl)-2-methyl-1-(4-methylpiperazin-1-yl)-pentan-3-ol
3-(4-chloro-3-trifluoromethylphenyl)-2-methyl-1-(4-methylpiperazin-1-yl)-pentan-3-ol
3-(3-fluorobenzyl)-2-methyl-1-(4-methylpiperazin-1-yl)-pentan-3-ol
3-(2-methoxyphenyl)-2-methyl-1-(4-methylpiperazin-1-yl)-pentan-3-ol
2-methyl-3-(2-methylbenzyl)-1-(4-methylpiperazin-1-yl)-pentan-3-ol
3-(3-chloro-4-fluorophenyl)-2-methyl-1-(4-methylpiperazin-1-yl)-pentan-3-ol
2-methyl-1-(4-methylpiperazin-1-yl)-3-(3-trifluoromethylphenyl)-pentan-3-ol
2-methyl-3-(3-methylbenzyl)-1-(4-methylpiperazin-1-yl)-pentan-3-ol
3-(4-chlorobenzyl)-2-methyl-1-(4-methylpiperazin-1-yl)-pentan-3-ol
3-(2-chloro-6-fluorobenzyl)-2-methyl-1-(4-methylpiperazin-1-yl)-pentan-3-ol
3-(2,5-dimethylbenzyl)-2-methyl-1-(4-methylpiperazin-1-yl)-pentan-3-ol
3-(3-chlorobenzyl)-2-methyl-1-(4-methylpiperazin-1-yl)-pentan-3-ol
3-(2,4-dichlorobenzyl)-2-methyl-1-(4-methylpiperazin-1-yl)-pentan-3-ol
1-cyclohexylmethyl-2-(4-methylpiperazin-1-ylmethyl)-cyclohexanol
1-(5-fluoro-2-methoxyphenyl)-2-(4-methylpiperazin-1-ylmethyl)-cyclohexanol
1-(3-fluorophenyl)-2-(4-methylpiperazin-1-ylmethyl)-cyclohexanol
1-(3-chlorophenyl)-2-(4-methylpiperazin-1-ylmethyl)-cyclohexanol
1-(3,5-dichlorophenyl)-2-(4-methylpiperazin-1-ylmethyl)-cyclohexanol
1-(2-chlorobenzyl)-2-(4-methylpiperazin-1-ylmethyl)-cyclohexanol
1-(4-fluorobenzyl)-2-(4-methylpiperazin-1-ylmethyl)-cyclohexanol
1-(3-methoxybenzyl)-2-(4-methylpiperazin-1-ylmethyl)-cyclohexanol
1-(4-chloro-3-trifluoromethylphenyl)-2-(4-methylpiperazin-1-ylmethyl)-cyclohexanol
1-(3-fluorobenzyl)-2-(4-methylpiperazin-1-ylmethyl)-cyclohexanol
1-(2-methoxyphenyl)-2-(4-methylpiperazin-1-ylmethyl)-cyclohexanol
1-(2-methylbenzyl)-2-(4-methylpiperazin-1-ylmethyl)-cyclohexanol
1-(3-chloro-4-fluorophenyl)-2-(4-methylpiperazin-1-ylmethyl)-cyclohexanol
2-(4-methylpiperazin-1-ylmethyl)-1-(3-trifluoromethylphenyl)-cyclohexanol
1-(3-methylbenzyl)-2-(4-methylpiperazin-1-ylmethyl)-cyclohexanol
1-(4-chlorobenzyl)-2-(4-methylpiperazin-1-ylmethyl)-cyclohexanol
1-(2,5-dimethylbenzyl)-2-(4-methylpiperazin-1-ylmethyl)-cyclohexanol
1-(3-chlorobenzyl)-2-(4-methylpiperazin-1-ylmethyl)-cyclohexanol
1-(2,4-dichlorobenzyl)-2-(4-methylpiperazin-1-ylmethyl)-cyclohexanol
1-cyclohexylmethyl-2-(4-methylpiperazin-1-ylmethyl)-cyclooctanol
1-(5-fluoro-2-methoxyphenyl)-2-(4-methylpiperazin-1-ylmethyl)-cyclooctanol
1-(2-chlorobenzyl)-2-(4-methylpiperazin-1-ylmethyl)-cyclooctanol
1-(4-fluorobenzyl)-2-(4-methylpiperazin-1-ylmethyl)-cyclooctanol
1-(3-fluorobenzyl)-2-(4-methylpiperazin-1-ylmethyl)-cyclooctanol
1-(2-methoxyphenyl)-2-(4-methylpiperazin-1-ylmethyl)-cyclooctanol
1-(3-methylbenzyl)-2-(4-methylpiperazin-1-ylmethyl)-cyclooctanol
1-(4-chlorobenzyl)-2-(4-methylpiperazin-1-ylmethyl)-cyclooctanol
1-(2-chloro-6-fluorobenzyl)-2-(4-methylpiperazin-1-ylmethyl)-cyclooctanol
1-(2,5-dimethylbenzyl)-2-(4-methylpiperazin-1-ylmethyl)-cyclooctanol
1-(3-chlorobenzyl)-2-(4-methylpiperazin-1-ylmethyl)-cyclooctanol
1-(2,4-dichlorobenzyl)-2-(4-methylpiperazin-1-ylmethyl)-cyclooctanol
1-cyclohexylmethyl-2-(4-methylpiperazin-1-ylmethyl)-cycloheptanol
1-(5-fluoro-2-methoxyphenyl)-2-(4-methylpiperazin-1-ylmethyl)-cycloheptanol
1-(3-fluorophenyl)-2-(4-methylpiperazin-1-ylmethyl)-cycloheptanol
1-(3-chlorophenyl)-2-(4-methylpiperazin-1-ylmethyl)-cycloheptanol
1-(3,5-dichlorophenyl)-2-(4-methylpiperazin-1-ylmethyl)-cycloheptanol
1-(2-chlorobenzyl)-2-(4-methylpiperazin-1-ylmethyl)-cycloheptanol
1-(4-fluorobenzyl)-2-(4-methylpiperazin-1-ylmethyl)-cycloheptanol
1-(3-methoxybenzyl)-2-(4-methylpiperazin-1-ylmethyl)-cycloheptanol
1-(4-chloro-3-trifluoromethylphenyl)-2-(4-methylpiperazin-1-ylmethyl)-cycloheptanol
1-(3-fluorobenzyl)-2-(4-methylpiperazin-1-ylmethyl)-cycloheptanol
1-(2-methoxyphenyl)-2-(4-methylpiperazin-1-ylmethyl)-cycloheptanol
1-(2-methylbenzyl)-2-(4-methylpiperazin-1-ylmethyl)-cycloheptanol
1-(3-chloro-4-fluorophenyl)-2-(4-methylpiperazin-1-ylmethyl)-cycloheptanol
2-(4-methylpiperazin-1-ylmethyl)-1-(3-trifluoromethylphenyl)-cycloheptanol
1-(3-methylbenzyl)-2-(4-methylpiperazin-1-ylmethyl)-cycloheptanol
1-(4-chlorobenzyl)-2-(4-methylpiperazin-1-ylmethyl)-cycloheptanol
1-(2-chloro-6-fluorobenzyl)-2-(4-methylpiperazin-1-ylmethyl)-cycloheptanol
1-(2,5-dimethylbenzyl)-2-(4-methylpiperazin-1-ylmethyl)-cycloheptanol
1-(3-chlorobenzyl)-2-(4-methylpiperazin-1-ylmethyl)-cycloheptanol
1-(2,4-dichlorobenzyl)-2-(4-methylpiperazin-1-ylmethyl)-cycloheptanol
1-benzyl-2-(4-methylpiperazin-1-ylmethyl)-cyclododecanol
2-(4-methylpiperazin-1-ylmethyl)-1-vinyl-cyclododecanol
1-cyclopentyl-2-(4-methylpiperazin-1-ylmethyl)-cyclododecanol
2-(4-methylpiperazin-1-ylmethyl)-1-phenethylcyclododecanol
2-(4-methylpiperazin-1-ylmethyl)-1-phenylethynylcyclododecanol
2-(4-methylpiperazin-1-ylmethyl)-1-thiophen-2-ylcyclododecanol
2-(4-methylpiperazin-1-ylmethyl)-1-(3-phenylpropyl)-cyclododecanol
1-(5-fluoro-2-methoxyphenyl)-2-(4-methylpiperazin-1-ylmethyl)-cyclododecanol
1-(2-chlorobenzyl)-2-(4-methylpiperazin-1-ylmethyl)-cyclododecanol
1-(4-fluorobenzyl)-2-(4-methylpiperazin-1-ylmethyl)-cyclododecanol
1-(3-methoxybenzyl)-2-(4-methylpiperazin-1-ylmethyl)-cyclododecanol
1-(3-fluorobenzyl)-2-(4-methylpiperazin-1-ylmethyl)-cyclododecanol
1-(2-methoxyphenyl)-2-(4-methylpiperazin-1-ylmethyl)-cyclododecanol
1-(2-methylbenzyl)-2-(4-methylpiperazin-1-ylmethyl)-cyclododecanol
1-(3-methylbenzyl)-2-(4-methylpiperazin-1-ylmethyl)-cyclododecanol
1-(4-chlorobenzyl)-2-(4-methylpiperazin-1-ylmethyl)-cyclododecanol
1-(2,5-dimethylbenzyl)-2-(4-methylpiperazin-1-ylmethyl)-cyclododecanol
1-(3-chlorobenzyl)-2-(4-methylpiperazin-1-ylmethyl)-cyclododecanol
1-(2,4-dichlorobenzyl)-2-(4-methylpiperazin-1-ylmethyl)-cyclododecanol
2-[4-(3-chlorophenyl)-piperazin-1-ylmethyl]-1-cyclohexylmethyl-cyclohexanol
2-[4-(3-chlorophenyl)-piperazin-1-ylmethyl]-1-(5-fluoro-2-methoxyphenyl)-cyclohexanol
2-[4-(3-chlorophenyl)-piperazin-1-ylmethyl]-1-(3-fluorophenyl)-cyclohexanol
1-(3-chlorophenyl)-2-[4-(3-chlorophenyl)-piperazin-1-ylmethyl]-cyclohexanol
2-[4-(3-chlorophenyl)-piperazin-1-ylmethyl]-1-(3,5-dichlorophenyl)-cyclohexanol
1-(2-chlorobenzyl)-2-[4-(3-chlorophenyl)-piperazin-1-ylmethyl]-cyclohexanol
2-[4-(3-chlorophenyl)-piperazin-1-ylmethyl]-1-(4-fluorobenzyl)-cyclohexanol
2-[4-(3-chlorophenyl)-piperazin-1-ylmethyl]-1-(4-chloro-3-trifluoromethylphenyl)-cyclohexanol
2-[4-(3-chlorophenyl)-piperazin-1-ylmethyl]-1-(3-fluorobenzyl)-cyclohexanol
2-[4-(3-chlorophenyl)-piperazin-1-ylmethyl]-1-(2-methoxyphenyl)-cyclohexanol
1-(3-chloro-4-fluorophenyl)-2-[4-(3-chlorophenyl)-piperazin-1-ylmethyl]-cyclohexanol
2-[4-(3-chlorophenyl)-piperazin-1-ylmethyl]-1-(3-trifluoromethylphenyl)-cyclohexanol
2-[4-(3-chlorophenyl)-piperazin-1-ylmethyl]-1-(3-methylbenzyl)-cyclohexanol
1-(4-chlorobenzyl)-2-[4-(3-chlorophenyl)-piperazin-1-ylmethyl]-cyclohexanol
1-(2-chloro-6-fluorobenzyl)-2-[4-(3-chlorophenyl)-piperazin-1-ylmethyl]-cyclohexanol
2-[4-(3-chlorophenyl)-piperazin-1-ylmethyl]-1-(2,5-dimethylbenzyl)-cyclohexanol
1-(3-chlorobenzyl)-2-[4-(3-chlorophenyl)-piperazin-1-ylmethyl]-cyclohexanol
2-[4-(2-chlorophenyl)-piperazin-1-ylmethyl]-1-phenylcyclohexanol
1-(4-chlorophenyl)-2-[4-(2-chlorophenyl)-piperazin-1-ylmethyl]-cyclohexanol
1-benzyl-2-[4-(2-chlorophenyl)-piperazin-1-ylmethyl]-cyclohexanol
2-[4-(2-chlorophenyl)-piperazin-1-ylmethyl]-1-(4-fluoro-3-methylphenyl)-cyclohexanol
2-[4-(2-chlorophenyl)-piperazin-1-ylmethyl]-1-o-tolyl-cyclohexanol
2-[4-(2-chlorophenyl)-piperazin-1-ylmethyl]-1-vinylcyclohexanol
1-(4-tert-butylphenyl)-2-[4-(2-chlorophenyl)-piperazin-1-ylmethyl]-cyclohexanol
2-[4-(2-chlorophenyl)-piperazin-1-ylmethyl]-1-cyclopentyl-cyclohexanol
2-[4-(2-chlorophenyl)-piperazin-1-ylmethyl]-1-m-tolyl-cyclohexanol
2-[4-(2-chlorophenyl)-piperazin-1-ylmethyl]-bicyclohexyl-1-ol
2-[4-(2-chlorophenyl)-piperazin-1-ylmethyl]-1-(4-fluorophenyl)-cyclohexanol
2-[4-(2-chlorophenyl)-piperazin-1-ylmethyl]-1-phenethyl-cyclohexanol
2-[4-(2-chlorophenyl)-piperazin-1-ylmethyl]-1-phenylethynyl-cyclohexanol
2-[4-(2-chlorophenyl)-piperazin-1-ylmethyl]-1-thiophen-2-yl-cyclohexanol
2-[4-(2-chlorophenyl)-piperazin-1-ylmethyl]-1-(3-methoxyphenyl)-cyclohexanol
2-[4-(2-chlorophenyl)-piperazin-1-ylmethyl]-1-(3-phenylpropyl)-cyclohexanol
2-[4-(2-chlorophenyl)-piperazin-1-ylmethyl]-1-p-tolyl-cyclohexanol
2-[4-(2-chlorophenyl)-piperazin-1-ylmethyl]-1-(4-methoxyphenyl)-cyclohexanol
2-[4-(2-chlorophenyl)-piperazin-1-ylmethyl]-1-cyclohexylmethyl-cyclohexanol
2-[4-(2-chlorophenyl)-piperazin-1-ylmethyl]-1-(5-fluoro-2-methoxyphenyl)-cyclohexanol
1-(3-chlorophenyl)-2-[4-(2-chlorophenyl)-piperazin-1-ylmethyl]-cyclohexanol
2-[4-(2-chlorophenyl)-piperazin-1-ylmethyl]-1-(3,5-dichlorophenyl)-cyclohexanol
1-(2-chlorobenzyl)-2-[4-(2-chlorophenyl)-piperazin-1-ylmethyl]-cyclohexanol
2-[4-(2-chlorophenyl)-piperazin-1-ylmethyl]-1-(4-fluorobenzyl)-cyclohexanol
2-[4-(2-chlorophenyl)-piperazin-1-ylmethyl]-1-(3-methoxybenzyl)-cyclohexanol
2-[4-(2-chlorophenyl)-piperazin-1-ylmethyl]-1-(4-chloro-3-trifluoromethylphenyl)-cyclohexanol
2-[4-(2-chlorophenyl)-piperazin-1-ylmethyl]-1-(3-fluorobenzyl)-cyclohexanol
2-[4-(2-chlorophenyl)-piperazin-1-ylmethyl]-1-(2-methoxyphenyl)-cyclohexanol
2-[4-(2-chlorophenyl)-piperazin-1-ylmethyl]-1-(2-methylbenzyl)-cyclohexanol
1-(3-chloro-4-fluorophenyl)-2-[4-(2-chlorophenyl)-piperazin-1-ylmethyl]-cyclohexanol
2-[4-(2-chlorophenyl)-piperazin-1-ylmethyl]-1-(3-trifluoromethylphenyl)-cyclohexanol
2-[4-(2-chlorophenyl)-piperazin-1-ylmethyl]-1-(3-methylbenzyl)-cyclohexanol
1-(4-chlorobenzyl)-2-[4-(2-chlorophenyl)-piperazin-1-ylmethyl]-cyclohexanol
1-(2-chloro-6-fluorobenzyl)-2-[4-(2-chlorophenyl)-piperazin-1-ylmethyl]-cyclohexanol
2-[4-(2-chlorophenyl)-piperazin-1-ylmethyl]-1-(2,5-dimethylbenzyl)-cyclohexanol
1-(3-chlorobenzyl)-2-[4-(2-chlorophenyl)-piperazin-1-ylmethyl]-cyclohexanol
2-[4-(2-chlorophenyl)-piperazin-1-ylmethyl]-1-(2,4-dichlorobenzyl)-cyclohexanol
2-[4-(2-methoxyphenyl)-piperazin-1-ylmethyl]-1-phenylcyclohexanol
1-(4-chlorophenyl)-2-[4-(2-methoxyphenyl)-piperazin-1-ylmethyl]-cyclohexanol
1-benzyl-2-[4-(2-methoxyphenyl)-piperazin-1-ylmethyl]-cyclohexanol
1-(4-fluoro-3-methylphenyl)-2-[4-(2-methoxyphenyl)-piperazin-1-ylmethyl]-cyclohexanol
2-[4-(2-methoxyphenyl)-piperazin-1-ylmethyl]-1-o-tolyl-cyclohexanol
2-[4-(2-methoxyphenyl)-piperazin-1-ylmethyl]-1-vinylcyclohexanol
1-(4-tert-butylphenyl)-2-[4-(2-methoxyphenyl)-piperazin-1-ylmethyl]-cyclohexanol
1-cyclopentyl-2-[4-(2-methoxyphenyl)-piperazin-1-ylmethyl]-cyclohexanol
2-[4-(2-methoxyphenyl)-piperazin-1-ylmethyl]-1-m-tolyl-cyclohexanol
2-[4-(2-methoxyphenyl)-piperazin-1-ylmethyl]-bicyclohexyl-1-ol
1-(4-fluorophenyl)-2-[4-(2-methoxyphenyl)-piperazin-1-ylmethyl]-cyclohexanol
2-[4-(2-methoxyphenyl)-piperazin-1-ylmethyl]-1-phenethyl-cyclohexanol
2-[4-(2-methoxyphenyl)-piperazin-1-ylmethyl]-1-phenylethynyl-cyclohexanol
2-[4-(2-methoxyphenyl)-piperazin-1-ylmethyl]-1-thiophen-2-yl-cyclohexanol
1-(3-methoxyphenyl)-2-[4-(2-methoxyphenyl)-piperazin-1-ylmethyl]-cyclohexanol
2-[4-(2-methoxyphenyl)-piperazin-1-ylmethyl]-1-(3-phenylpropyl)-cyclohexanol
1-(2,3-dichlorophenyl)-2-[4-(2-methoxyphenyl)-piperazin-1-ylmethyl]-cyclohexanol
2-[4-(2-methoxyphenyl)-piperazin-1-ylmethyl]-1-p-tolyl-cyclohexanol
1-(4-methoxyphenyl)-2-[4-(2-methoxyphenyl)-piperazin-1-ylmethyl]-cyclohexanol
1-cyclohexylmethyl-2-[4-(2-methoxyphenyl)-piperazin-1-ylmethyl]-cyclohexanol
1-(5-fluoro-2-methoxyphenyl)-2-[4-(2-methoxyphenyl)-piperazin-1-ylmethyl]-cyclohexanol
1-(3-fluorophenyl)-2-[4-(2-methoxyphenyl)-piperazin-1-ylmethyl]-cyclohexanol
1-(3-chlorophenyl)-2-[4-(2-methoxyphenyl)-piperazin-1-ylmethyl]-cyclohexanol
1-(3,5-dichlorophenyl)-2-[4-(2-methoxyphenyl)-piperazin-1-ylmethyl]-cyclohexanol
1-(2-chlorobenzyl)-2-[4-(2-methoxyphenyl)-piperazin-1-ylmethyl]-cyclohexanol
1-(4-fluorobenzyl)-2-[4-(2-methoxyphenyl)-piperazin-1-ylmethyl]-cyclohexanol
1-(3-methoxybenzyl)-2-[4-(2-methoxyphenyl)-piperazin-1-ylmethyl]-cyclohexanol
1-(4-chloro-3-trifluoromethylphenyl)-2-[4-(2-methoxyphenyl)-piperazin-1-ylmethyl]-cyclohexanol
1-(3-fluorobenzyl)-2-[4-(2-methoxyphenyl)-piperazin-1-ylmethyl]-cyclohexanol
1-(2-methoxyphenyl)-2-[4-(2-methoxyphenyl)-piperazin-1-ylmethyl]-cyclohexanol
2-[4-(2-methoxyphenyl)-piperazin-1-ylmethyl]-1-(2-methylbenzyl)-cyclohexanol
1-(3-chloro-4-fluorophenyl)-2-[4-(2-methoxyphenyl)-piperazin-1-ylmethyl]-cyclohexanol
2-[4-(2-methoxyphenyl)-piperazin-1-ylmethyl]-1-(3-trifluoromethylphenyl)-cyclohexanol
2-[4-(2-methoxyphenyl)-piperazin-1-ylmethyl]-1-(3-methylbenzyl)-cyclohexanol
1-(4-chlorobenzyl)-2-[4-(2-methoxyphenyl)-piperazin-1-ylmethyl]-cyclohexanol
1-(2-chloro-6-fluorobenzyl)-2-[4-(2-methoxyphenyl)-piperazin-1-ylmethyl]-cyclohexanol
1-(2,5-dimethylbenzyl)-2-[4-(2-methoxyphenyl)-piperazin-1-ylmethyl]-cyclohexanol
1-(3-chlorobenzyl)-2-[4-(2-methoxyphenyl)-piperazin-1-ylmethyl]-cyclohexanol
1-(2,4-dichlorobenzyl)-2-[4-(2-methoxyphenyl)-piperazin-1-ylmethyl]-cyclohexanol
1-benzyl-2-[4-(2-methoxyphenyl)-piperazin-1-ylmethyl]-cyclododecanol
2-[4-(2-methoxyphenyl)-piperazin-1-ylmethyl]-1-vinyl-cyclododecanol
1-phenethyl-2-[phenyl-(4-phenyl-piperazin-1-yl)-methyl]-cyclohexanol
1-benzyl-2-[(4-methylpiperazin-1-yl)-phenylmethyl]-cyclohexanol
1-benzyl-2-[phenyl-(4-phenyl-piperazin-1-yl)-methyl]-cyclohexanol
2-[(4-methylpiperazin-1-yl)-phenylmethyl]-1-phenethyl-cyclohexanol
2-[4-(2-methoxyphenyl)-piperazin-1-ylmethyl]-1-phenethyl-cyclododecanol
in the form of the diastereomer, the enantiomer and a mixture thereof - including the racemate - and in the form of the corresponding base, the corresponding salt and the corresponding solvate.

## Revendications

1. Utilisation d'au moins un composé de pipérazine N,N'-disubstituée de formule générale I, 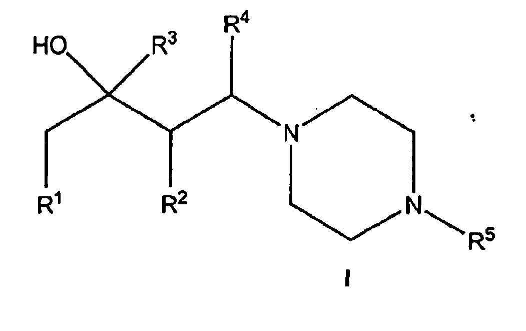 dans laquelle
R¹ et R ² ,identiques ou différents, représentent chacun un radical aliphatique saturé ou insaturé, linéaire ou ramifié, ou forment ensemble une chaîne (CH₂)ₙ, n représentant un nombre entier,
R³ et R⁵, identiques ou différents, représentent chacun un radical aliphatique saturé ou insaturé, linéaire ou ramifié, un radical cycloaliphatique saturé ou insaturé, un radical aryle ou un radical hétéroaryle, les systèmes cycliques respectifs étant éventuellement substitués une ou plusieurs fois et/ou étant reliés par un pont aliphatique saturé ou insaturé, linéaire ou ramifié et/ou le radical aryle ou hétéroaryle formant une partie d'un système polycyclique,
R⁴ représente l'hydrogène ou un radical aryle ou hétéroaryle éventuellement substitué une ou plusieurs fois, le radical aryle ou hétéroaryle pouvant former une partie d'un système polycyclique,
sous forme de ses diastéréoisomères, de ses énantiomères et de leurs mélanges, y compris leurs racémates, ainsi que sous forme des bases, sels et solvates correspondants, en tant que substance active pour la fabrication d'un médicament pour le traitement de
• l'épilepsie,
• la douleur neuropathique, chronique et/ou aiguë,
• les symptômes associés à la douleur neuropathique,
• la migraine,
• l'hyperalgésie, notamment l'hyperalgésie thermique
• et/ou l'hyperalgésie mécanique,
• l'allodynie, notamment l'allodynie mécanique et/ou l'allodynie au froid,
• la douleur inflammatoire,
• la douleur post-opératoire,
• les maladies neurodégénératives, notamment la maladie d'Alzheimer, la maladie d'Huntington et/ou la maladie de Parkinson,
• les troubles psychiatriques et/ou les troubles neuropathologiques, notamment les troubles bipolaires, l'anxiété, les crises de panique, les troubles de l'humeur, les comportements maniaques et/ou les comportements maniaco-dépressifs,
• les bouffées de chaleur,
• les troubles post-ménoposiques,
• la sclérose latérale amyotrophique (SLA),
• la dystrophie sympathique réflexe (DSR),
• la paralysie spastique,
• le syndrome des jambes sans repos,
• le nystagmus acquis,
• la neuropathie diabétique douloureuse,
• les symptômes et/ou les douleurs liées à la sclérose en plaque et/ou à la maladie de Parkinson
• les perturbations gastro-intestinales,
• la douleur érythromélalgique,
• la douleur postpoliomyélitique,
• la névralgie trigéminale et/ou
• la névralgie post-herpétique.

2. Utilisation selon la revendication 1,
**caractérisée en ce que**
R¹ et R², identiques ou différents, représentent chacun un radical en C₁₋₆ aliphatique saturé ou insaturé, linéaire ou ramifié, ou forment ensemble une chaîne (CH₂)ₙ, n représentant un nombre entier compris entre 2 et 9,
R³ représente un radical en C₁₋₆ aliphatique, saturé ou insaturé, linéaire ou ramifié, un radical en C₃₋₇ cycloaliphatique saturé ou insaturé, un radical phényle ou un radical hétéroaryle à cinq ou six éléments, les systèmes cycliques respectifs étant éventuellement substitués une ou plusieurs fois et/ou étant reliés par un pont en C₁₋₅ aliphatique saturé ou insaturé, linéaire ou ramifié,
R⁴ représente l'hydrogène ou un radical phényle,
R⁵ représente un radical en C₁₋₆ aliphatique saturé ou insaturé, linéaire ou ramifié ou un radical phényle éventuellement substitué.

3. Utilisation selon la revendication 1, **caractérisée en ce que**
R¹ et R², identiques ou différents, représentent chacun un radical en C₁₋₃ aliphatique saturé ou insaturé, linéaire ou ramifié, ou forment ensemble une chaîne (CH₂)ₙ, n représentant un nombre entier compris entre 2 et 9,
R³ représente un radical en C₁₋₃ aliphatique, saturé ou insaturé, linéaire ou ramifié, un radical en C₅₋₆ cycloaliphatique saturé ou insaturé, un radical phényle ou un radical hétéroaryle à cinq ou six éléments, les systèmes cycliques respectifs étant éventuellement substitués une ou plusieurs fois par un halogène, un groupement alkyle, un groupement alcoxy et/ou un groupement alkyle trihalogéné et/ou étant reliés par un pont en C₁₋₃ aliphatique saturé ou insaturé, linéaire ou ramifié,
R⁴ représente l'hydrogène ou un radical phényle,
R⁵ représente un radical en C₁₋₃ aliphatique saturé ou insaturé, linéaire ou ramifié ou un radical phényle éventuellement substitué par un halogène ou un groupement alcoxy.

4. Utilisation selon la revendication 1, **caractérisée en ce que**
R¹ et R² représentent chacun un groupement méthyle ou forment ensemble une chaîne (CH₂)ₙ, n représentant 3, 4, 5 ou 9.
R³ représente un radical vinyle, un radical cyclopentyle, un radical cyclohexyle, un radical thiophényle ou un radical phényle, le radical cyclohexyle étant éventuellement relié par un pont méthylène ou le radical phényle étant éventuellement substitué une ou plusieurs fois par du fluor, du chlore, un groupement méthyle, un groupement isopropyle, un groupement méthoxy et/ou un groupement trifluorométhyle et/ou étant éventuellement relié par un pont en C₁₋₃ aliphatique linéaire saturé ou un pont éthynyle,
R⁴ représente l'hydrogène ou un radical phényle,
R⁵ représente un groupement méthyle ou un radical phényle éventuellement substitué par du chlore ou un groupement méthoxy.

5. Utilisation selon la revendication 1 du composé de formule générale II

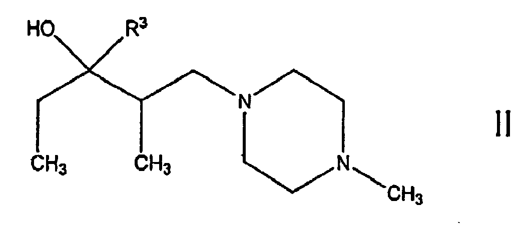

6. Utilisation selon la revendication 1 du composé de formule générale III, 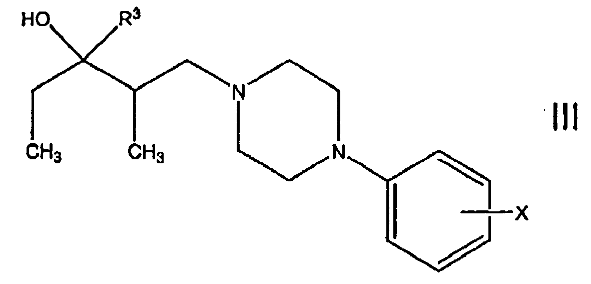 dans laquelle
X représente l'hydrogène, un halogène ou un groupement alcoxy, de préférence l'hydrogène, le chlore ou un groupement méthoxy.

7. Utilisation selon la revendication 1 du composé de formule générale IV, 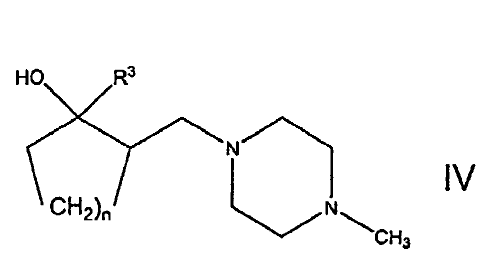 dans laquelle
n représente un nombre entier compris entre 2 et 9.

8. Utilisation selon la revendication 1 du composé de formule générale V, 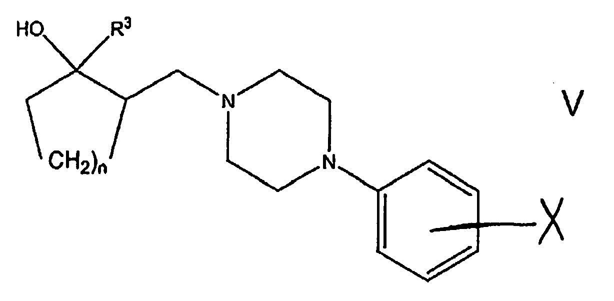 dans laquelle
n représente un nombre entier compris entre 2 et 9,
X représente l'hydrogène, un halogène ou un groupement alcoxy, de préférence l'hydrogène, le chlore ou un groupement méthoxy.

9. Utilisation selon la revendication 1 du composé de formule générale VI, 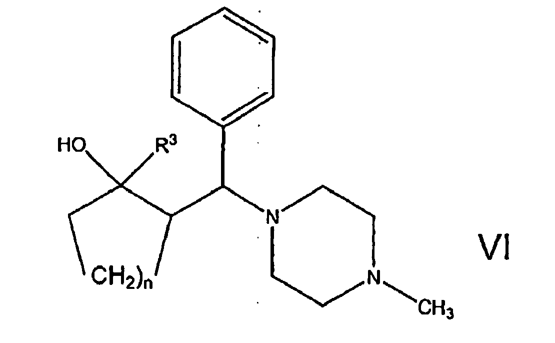 dans laquelle
n représente un nombre entier compris entre 2 et 9.

10. Utilisation selon la revendication 1 du composé de formule générale VII, 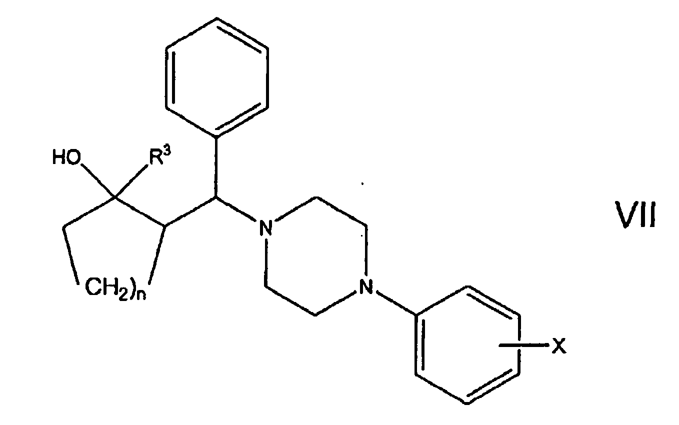 dans laquelle
n représente un nombre entier compris entre 2 et 9,
X représente l'hydrogène, un halogène ou un groupement alcoxy, de préférence l'hydrogène, le chlore ou un groupement méthoxy.

11. Utilisation selon l'une quelconque des revendications 5 à 10, **caractérisée en ce que**
R³ représente un radical en C₁₋₆ aliphatique, saturé ou insaturé, linéaire ou ramifié, un radical en C₃₋₇ cycloaliphatique saturé ou insaturé, un radical phényle ou un radical hétéroaryle à cinq ou six éléments, les systèmes cycliques respectifs étant éventuellement substitués une ou plusieurs fois et/ou étant reliés par un pont en C₁₋₅ aliphatique saturé ou insaturé, linéaire ou ramifié.

12. Utilisation selon l'une quelconque des revendications 5 à 10, **caractérisée en ce que**
R³ représente un radical en C₁₋₃ aliphatique, saturé ou insaturé, linéaire ou ramifié, un radical en C₅₋₆ cycloaliphatique saturé ou insaturé, un radical phényle ou un radical hétéroaryle à cinq ou six éléments, les systèmes cycliques respectifs étant éventuellement substitués une ou plusieurs fois avec un halogène, un groupement alkyle, un groupement alcoxy et/ou un groupement alkyle trihalogéné et/ou étant reliés par un pont en C₁₋₃ aliphatique saturé ou insaturé, linéaire ou ramifié,

13. Utilisation selon l'une quelconque des revendications 5 à 10, **caractérisée en ce que**
R³ représente un radical vinyle, un radical cyclopentyle, un radical cyclohexyle, un radical thiophényle ou un radical phényle, le radical cyclohexyle étant éventuellement relié par un pont méthylène ou le radical phényle étant éventuellement substitué une ou plusieurs fois par du fluor, du chlore, un groupement méthyle, un groupement isopropyle, un groupement méthoxy et/ou un groupement trifluorométhyle et/ou étant éventuellement relié par un pont en C₁₋₃ aliphatique linéaire saturé ou un pont éthynyle,

14. Utilisation selon la revendication 1, le composé de pipérazine N,N'-disubstituée étant choisi parmi
2-Méthyl-1-(4-méthyl-pipérazin-1-yl)-3-phényl-pentan-3-ol
3-(4-Chloro-phényl)-2-méthyl-1-(4-méthyl-pipérazin-1-yl)-pentan-3-ol
3-Benzyl-2-méthyl-1-(4-méthyl-pipérazin-1-yl)-pentan-3-ol
3-(4-Fluoro-3-méthyl-phényl)-2-méthyl-1-(4-méthylpipérazin-1-yl)-pentan-3-ol
2-Méthyl-1-(4-méthyl-pipérazin-1-yl)-3-o-tolyl-pentan-3-ol
3-Éthyl-4-méthyl-5-(4-méthyl-pipérazin-1-yl)-pent-1-én-3-ol
3-4-tert-Butyl-phényl)-2-méthyl-1-(4-méthyl-pipérazin-1-yl)-pentan-3-ol
3-Cyclopentyl-2-méthyl-1-(4-méthyl-pipérazin-1-yl)-pentan-3-ol
2-Méthyl-1-(4-méthyl-pipérazin-1-yl)-3-m-tolyl-pentan-3-ol
3-Cyclohexyl-2-méthyl-1-(4-méthyl-pipérazin-1-yl)-pentan-3-ol
3-(4-Fluoro-phényl)-2-méthyl-1-(4-méthyl-pipérazin-1-yl)-pentan-3-ol
3-Éthyl-2-méthyl-1-(4-méthyl-pipérazin-1-yl)-5-phénylpentan-3-ol
3-Éthyl-4-méthyl-5- (4-méthyl-pi,pérazin-1-yl) -1-phénylpent-1-yn-3-ol
2-Méthyl-1-(4-méthyl-pipérazin-1-yl)-3-thiophén-2-ylpentan-3-ol
3-(3-Méthoxy-phényl)-2-méthyl-1-(4-méthyl-pipérazin-1-yl)-pentan-3-ol
3-Éthyl-2-méthyl-1-(4-méthyl-pipérazin-1-yl)-6-phénylhexan-3-ol
2-Méthyl-1-(4-méthyl-pipérazin-1-yl)-3-p-tolyl-pentan-3-ol
3-(4-Méthoxy-phényl)-2-méthyl-1-(4-méthyl-pipérazin-1-yl)-pentan-3-ol
2-(4-Méthyl-pipérazin-1-ylméthyl)-1-phényl-cyclohexanol
1-Benzyl-2-(4-méthyl-pipérazin-1-ylméthyl)-cyclohexanol
1-(4-Fluoro-3-méthyl-phényl)-2-(4-méthyl-pipérazin-1ylméthyl)-cyclohexanol
2-(4-Méthyl-pipérazin-1-ylméthyl)-1-o-tolyl-cyclohexanol
2-(4-Méthyl-pipérazin-1-ylméthyl)-1-vinyl-cyclohexanol
1-Cyclopentyl-2-(4-méthyl-pipérazin-1-ylméthyl)-cyclohexanol
2-(4-Méthyl-pipérazin-1-ylméthyl)-1-m-tolyl-cyclohexanol
2-(4-Méthyl-pipérazin-1-ylméthyl)-bicyclohexyl-1-ol
1-(4-Fluoro-phényl)-2-(4-méthyl-pipérazin-1-ylméthyl)-cyclohexanol
2-(4-Méthyl-pipérazin-1-ylméthyl)-1-phénéthylcyclohexanol
2-(4-Méthyl-pipérazin-1-ylméthyl)-1-thiophén-2-ylcyclohexanol
1-(2,4-Dichloro-phényl)-2-(4-méthyl-pipérazin-1-ylméthyl)-cyclohexanol
1-(3-Méthoxy-phényl)-2-(4-méthyl-pipérazin-1-ylméthyl)-cyclohexanol
2-(4-Méthyl-pipérazin-1-ylméthyl)-1-(3-phényl-propyl)-cyclohexanol
2-(4-Méthyl-pipérazin-1-ylméthyl)-1-p-tolyl-cyclohexanol
1-(4-Méthoxy-phényl)-2-(4-méthyl-pipérazin-1-ylméthyl)-cyclohexanol
1-Benzyl-2-(4-méthyl-pipérazin-1-ylméthyl)-cyclooctanol
2-(4-Méthyl-pipérazin-1-ylméthyl)-1-vinyl-cyclooctanol
1-(4-tert-Butyl-phényl)-2-(4-méthyl-pipérazin-1-ylméthyl)-cyclooctanol
1-Cyclopentyl-2-(4-méthyl-pipérazin-1-ylméthyl)-cyclooctanol
2-(4-Méthyl-pipérazin-1-ylméthyl)-1-phénéthylcyclooctanol
2-(4-Méthyl-pipérazin-1-ylméthyl)-1-phényléthynylcyclooctanol
2-(4-Méthyl-pipérazin-1-ylméthyl)-1-thiophén-2-ylcyclooctanol
1-(3-Méthoxy-phényl)-2-(4-méthyl-pipérazin-1-ylméthyl)-cyclooctanol
2-(4-Méthyl-pipérazin-1-ylméthyl)-1-(3-phényl-propyl)-cyclooctanol
2-(4-Méthyl-pipérazin-1-ylméthyl)-1-p-tolylcyclooctanol
1-(4-Méthoxy-phényl)-2-(4-méthyl-pipérazin-1-ylméthyl)-cyclooctanol
2-(4-Méthyl-pipérazin-1-ylméthyl)-1-phénylcycloheptanol
1-(4-Chloro-phényl)-2-(4-méthyl-pipérazin-1-ylméthyl)-cycloheptanol
1-Benzyl-2-(4-méthyl-pipérazin-1-ylméthyl)-cycloheptanol
1-(4-Fluoro-3-méthyl-phényl)-2-(4-méthyl-pipérazin-1-ylméthyl)-cycloheptanol
2-(4-Méthyl-pipérazin-1-ylméthyl)-1-o-tolylcycloheptanol
2-(4-Méthyl-pipérazin-1-ylméthyl)-1-vinyl-cycloheptanol
1-(4-tert-Butyl-phényl)-2-(4-méthyl-pipérazin-1-ylméthyl)-cycloheptanol
1-Cyclopentyl-2-(4-méthyl-pipérazin-1-ylméthyl)-cycloheptanol
2-(4-Méthyl-pipérazin-1-ylméthyl)-1-m-tolylcycloheptanol
1-[4-3-Chloro-phényl)-pipérazin-1-yl]-2-méthyl-3-phényl-pentan-3-ol
3-(4-Chloro-phényl)-1-[4-(3-chloro-phényl)-pipérazin-1-yl]-2-méthyl-pentan-3-ol
3-Benzyl-1-[4-(3-chloro-phényl)-pipérazin-1-yl]-2-méthyl-pentan-3-ol
1-[4-(3-Chloro-phényl)-pipérazin-1-yl]-3-(4-fluoro-3-méthyl-phényl)-2-méthyl-pentan-3-ol
5-[4-(3-Chloro-phényl)-pipérazin-1-yl]-3-éthyl-4-méthyl-pent-1-én-3-ol
3-(4-tert-Butyl-phényl)-1-[4-(3-chloro-phényl)-pipérazin-1-yl]-2-méthyl-pentan-3-ol
1-[4-(3-Chloro-phényl)-pipérazin-1-yl]-2-méthyl-3-m-tolyl-pentan-3-ol
1-[4-(3-Chloro-phényl)-pipérazin-1-yl]-3-cyclohexyl-2-méthyl-pentan-3-ol
1-[4-(3-Chloro-phényl)-pipérazin-1-yl]-3-(4-fluorophényl)-2-méthyl-pentan-3-ol
1-[4-(3-Chloro-phényl)-pipérazin-1-yl]-3-éthyl-2-méthyl-5-phényl-pentan-3-ol
5-[4-(3-Chloro-phényl)-pipérazin-1-yl]-3-éthyl-4-méthyl-1-phényl-pent-1-yn-3-ol
1-[4-(3-Chloro-phényl)-pipérazin-1-yl]-2-méthyl-3-thiophén-2-yl-pentan-3-ol
1-[4-(3-Chloro-phényl)-pipérazin-1-yl]-3(3-méthoxyphényl)-2-méthyl-pentan-3-ol
1-[4-(3-Chloro-phényl)-pipérazin-1-yl]-3-éthyl-2-méthyl-6-phényl-hexan-3-ol
1-[4-(3-Chloro-phényl)-pipérazin-1-yl]-2-méthyl-3-p-tolyl-pentan-3-ol
1-[4-(3-Chloro-phényl)-pipérazin-1-yl]-3-(4-méthoxyphényl)-2-méthyl-pentan-3-ol
1-[4-(3-,Chloro-phényl)-pipérazin-1-yl]-3-(5-fluoro-2-méthoxy-phényl)-2-méthyl-pentan-3-ol
1-[4-(3-Chloro-phényl)-pipérazin-1-yl]-3-(3-fluorophényl)-2-méthyl-pentan-3-ol
3-(3-Chloro-phényl)-1-[4-(3-chloro-phényl)-pipérazin-1-yl]-2-méthyl-pentan-3-ol
3-(2-Chloro-benzyl)-1-[4-(3-chloro-phényl)-pipérazin-1-yl]-2-méthyl-pentan-3-ol
1-[4-(3-Chloro-phényl)-pipérazin-1-yl]-3-(4-fluorobenzyl)-2-méthyl-pentan-3-ol
1-[4-(3-Chloro-phényl)-pipérazin-1-yl]-3-(3-méthoxybenzyl)-2-méthyl-pentan-3-ol
1-[4-(3-Chloro-phényl)-pipérazin-1-yl]-3-(3-fluorobenzyl)-2-méthyl-pentan-3-ol
1-[4-(3-Chloro-phényl)-pipérazin-1-yl]-3-(2-méthoxyphényl)-2-méthyl-pentan-3-ol
1-[4-(3-Chloro-phényl)-pipérazin-1-yl]-2-méthyl-3-(2-méthyl-benzyl)-pentan-3-ol
1-[4-(3-Chloro-phényl)-pipérazin-1-yl]-2-méthyl-3-(3-trifluorométhyl-phényl)-pentan-3-ol
1-[4-(3-Chloro-phényl)-pipérazin-1-yl]-2-méthyl-3-(3-méthyl-benzyl)-pentan-3-ol
3-(4-Chloro-benzyl)-1-[4-(3-chloro-phényl)-pipérazin-1-yl]-2-méthyl-pentan-3-ol
3-(2-Chloro-6-fluoro-benzyl)-1-[4-(3-chloro-phényl)-pipérazin-1-yl]-2-méthyl-pentan-3-ol
1-[4-(3-Chloro-phényl)-pipérazin-1-yl]-3-(2,5-diméthylbenzyl)-2-méthyl-pentan-3-ol
3-(3-Chloro-benzyl)-1-[4-(3-chloro-phényl)-pipérazin-1-yl]-2-méthyl-pentan-3-ol
1-[4-(3-Chloro-phényl)-pipérazin-1-yl]-3-(2,4-dichlorobenzyl)-2-méthyl-pentan-3-ol
3-Cyclohexylméthyl-2-méthyl-1-(4-méthyl-pipérazin-1-yl)-pentan-3-ol
3-(5-Fluoro-2-méthoxy-phényl)-2-méthyl-1-(4-méthylpipérazin-1-yl)-pentan-3-ol
3-(3-Fluoro-phényl)-2-méthyl-1-(4-méthyl-pipérazin-1-yl)-pentan-3-ol
3-(3-Chloro-phényl)-2-méthyl-1-(4-méthyl-pipérazin-1-yl) -pentan-3-ol
3-(3,5-Dichloro-phényl)-2-méthyl-1-(4-méthyl-pipérazin-1-yl)-pentan-3-ol
3-(2-Chloro-benzyl)-2-méthyl-1-(4-méthyl-pipérazin-1-yl)-pentan-3-ol
3-(4-Fluoro-benzyl)-2-méthyl-1-(4-méthyl-pipérazin-1-yl)-pentan-3-ol
3-(3-Méthoxy-benzyl)-2-méthyl-1-(4-méthyl-pipérazin-1-yl) -pentan-3-ol
3-(4-Chloro-3-trifluorométhyl-phényl)-2-méthyl-1-(4-méthyl-pipérazin-1-yl)-pentan-3-ol
3-(3-Fluoro-benzyl)-2-méthyl-1-(4-méthyl-pipérazin-1-yl)-pentan-3-ol
3-(2-Méthoxy-phényl)-2-méthyl-1-(4-méthyl-pipérazin-1-yl)-pentan-3-ol
2-Méthyl-3-(2-méthyl-benzyl)-1-(4-méthyl-pipérazin-1-yl)-pentan-3-ol
3-(3-Chloro-4-fluoro-phényl)-2-méthyl-1-(4-méthylpipérazin-1-yl)-pentan-3-ol
2-Méthyl-1-(4-méthyl-pipérazin-1-yl)-3-(3-trifluorométhyl-phényl)-pentan-3-ol
2-Méthyl-3-(3-méthyl-benzyl)-1-(4-méthyl-pipérazin-1-yl)-pentan-3-ol
3-(4-Chloro-benzyl)-2-méthyl-1-(4-méthyl-pipérazin-1-yl) -pentan-3-ol
3-(2-Chloro-6-fluoro-benzyl)-2-méthyl-1-(4-méthylpipérazin-1-yl)-pentan-3-ol
3-(2,5-Diméthyl-benzyl)-2-méthyl-1-(4-méthyl-pipérazin-1-yl)-pentan-3-ol
3-(3-Chloro-benzyl)-2-méthyl-1-(4-méthyl-pipérazin-1-yl)-pentan-3-ol
3-(2,4-Dichloro-benzyl)-2-méthyl-1-(4-méthyl-pipérazin-1-yl)-pentan-3-ol
1-Cyclohexylméthyl-2-(4-méthyl-pipérazin-1-ylméthyl)-cyclohexanol
1-(5-Fluoro-2-méthoxy-phényl)-2-(4-méthyl-pipérazin-1-ylméthyl)-cyclohexanol
1-(3-Fluoro-phényl)-2-(4-méthyl-pipérazin-1-ylméthyl)-cyclohexanol
1-(3-Chloro-phényl)-2-(4-méthyl-pipérazin-1-ylméthyl)-cyclohexanol
1-(3,5-Dichloro-phényl)-2-(4-méthyl-pipérazin-1-ylméthyl)-cyclohexanol
1-(2-Chloro-benzyl)-2-(4-méthyl-pipérazin-1-ylméthyl)-cyclohexanol
1-(4-Fluoro-benzyl)-2-(4-méthyl-pipérazin-1-ylméthyl)-cyclohexanol
1-(3-Méthoxy-benzyl)-2-(4-méthyl-pipérazin-1-ylméthyl)-cyclohexanol
1-(4-Chloro-3-trifluorométhyl-phényl)-2-(4-méthylpipérazin-1-ylméthyl)-cyclohexanol
1-(3-Fluoro-benzyl)-2-(4-méthyl-pipérazin-1-ylméthyl)-cyclohexanol
1-(2-Méthoxy-phényl)-2-(4-méthyl-pipérazin-1-ylméthyl)-cyclohexanol
1-(2-Méthyl-benzyl)-2-(4-méthyl-pipérazin-1-ylméthyl)-cyclohexanol
1-(3-Chloro-4-fluoro-phényl)-2-(4-méthyl-pipérazin-1-ylméthyl)-cyclohexanol
2-(4-Méthyl-pipérazin-1-ylméthyl)-1-(3-trifluorométhylphényl)-cyclohexanol
1-(3-Méthyl-benzyl)-2-(4-méthyl-pipérazin-1-ylméthyl)-cyclohexanol
1-(4-Chloro-benzyl)-2-(4-méthyl-pipérazin-1-ylméthyl)-cyclohexanol
1-(2,5-Diméthyl-benzyl)-2-(4-méthyl-pipérazin-1-ylméthyl)-cyclohexanol
1-(3-Chloro-benzyl)-2-(4-méthyl-pipérazin-1-ylméthyl)-cyclohexanol
1-(2,4-Dichloro-benzyl)-2-(4-méthyl-pipérazin-1-ylméthyl)-cyclohexanol
1-Cyclohexylméthyl-2-(4-méthyl-pipérazin-1-ylméthyl)-cyclooctanol
1-(5-Fluoro-2-méthoxy-phényl)-2-(4-méthyl-pipérazin-1-ylméthyl)-cyclooctanol
1-(2-Chloro-benzyl)-2-(4-méthyl-pipérazin-1-ylméthyl)-cyclooctanol
1-(4-Fluoro-benzyl)-2-(4-méthyl-pipérazin-1-ylméthyl)-cyclooctanol
1-(3-Fluoro-benzyl)-2-(4-méthyl-pipérazin-1-ylméthyl)-cyclooctanol
1-(2-Méthoxy-phényl)-2-(4-méthyl-pipérazin-1-ylméthyl)-cyclooctanol
1-(3-Méthyl-benzyl)-2-(4-méthyl-pipérazin-1-ylméthyl)-cyclooctanol
1-(4-Chloro-benzyl)-2-(4-méthyl-pipérazin-1-ylméthyl)-cyclooctanol
1-(2-Chloro-6-fluoro-benzyl)-2-(4-méthyl-pipérazin-1-ylméthyl)-cyclooctanol
1-(2,5-Diméthyl-benzyl)-2-(4-méthyl-pipérazin-1-ylméthyl)-cyclooctanol
1-(3-Chloro-benzyl)-2-(4-méthyl-pipérazin-1-ylméthyl)-cyclooctanol
1-(2,4-Dichloro-benzyl)-2-(4-méthyl-pipérazin-1-ylméthyl)-cyclooctanol
1-Cyclohexylméthyl-2-(4-méthyl-pipérazin-1-ylméthyl)-cycloheptanol
1-(5-Fluoro-2-méthoxy-phényl)-2-(4-méthyl-pipérazin-1-ylméthyl)-cycloheptanol
1-(3-Fluoro-phényl)-2-(4-méthyl-pipérazin-1-ylméthyl)-cycloheptanol
1-(3-Chloro-phényl)-2-(4-méthyl-pipérazin-1-ylméthyl)-cycloheptanol
1-(3,5-Dichloro-phényl)-2-(4-méthyl-pipérazin-1-ylméthyl)-cycloheptanol
1-(2-Chloro-benzyl)-2-(4-méthyl-pipérazin-1-ylméthyl)-cycloheptanol
1-(4-Fluoro-benzyl)-2-(4-méthyl-pipérazin-1-ylméthyl)-cycloheptanol
1-(3-Méthoxy-benzyl)-2-(4-méthyl-pipérazin-1-ylméthyl)-cycloheptanol
1-(4-Chloro-3-trifluorométhyl-phényl)-2-(4-méthylpipérazin-1-ylméthyl)-cycloheptanol
1-(3-Fluoro-benzyl)-2-(4-méthyl-pipérazin-1-ylméthyl)-cycloheptanol
1-(2-Méthoxy-phényl)-2-(4-méthyl-pipérazin-1-ylméthyl)-cycloheptanol
1-(2-Méthyl-benzyl)-2-(4-méthyl-pipérazin-1-ylméthyl)-cycloheptanol
1-(3-Chloro-4-fluoro-phényl)-2-(4-méthyl-pipérazin-1-ylméthyl)-cycloheptanol
2-(4-Méthyl-pipérazin-1-ylméthyl)-1-(3-trifluorométhylphényl)-cycloheptanol
1-(3-Méthyl-benzyl)-2-(4-méthyl-pipérazin-1-ylméthyl)-cycloheptanol
1-(4-Chloro-benzyl)-2-(4-méthyl-pipérazin-1-ylméthyl)-cycloheptanol
1-(2-Chloro-6-fluoro-benzyl)-2-(4-méthyl-pipérazin-1-ylméthyl)-cycloheptanol
1-(2,5-Diméthyl-benzyl)-2-(4-méthyl-pipérazin-1-ylméthyl)-cycloheptanol
1-(3-Chloro-benzyl)-2-(4-méthyl-pipérazin-1-ylméthyl)-cycloheptanol
1-(2,4-Dichloro-benzyl)-2-(4-méthyl-pipérazin-1-ylméthyl)-cycloheptanol
1-Benzyl-2-(4-méthyl-pipérazin-1-ylméthyl)-cyclododécanol
2-(4-Méthyl-pipérazin-1-ylméthyl)-1-vinylcyclododécanol
1-Cyclopentyl-2-(4-méthyl-pipérazin-1-ylméthyl)-cyclododécanol
2-(4-Méthyl-pipérazin-1-ylméthyl)-1-phénéthylcyclododécanol
2-(4-Méthyl-pipérazin-1-ylméthyl)-1-phényléthynylcyclododécanol
2-(4-Méthyl-pipérazin-1-ylméthyl)-1-thiophén-2-ylcyclododécanol
2-(4-Méthyl-pipérazin-1-ylméthyl)-1-(3-phényl-propyl)-cyclododécanol
1-(5-Fluoro-2-méthoxy-phényl)-2-(4-méthyl-pipérazin-1-ylméthyl)-cyclododécanol
1-(2-Chloro-benzyl)-2-(4-méthyl-pipérazin-1-ylméthyl)-cyclododécanol
1-(4-Fluoro-benzyl)-2-(4-méthyl-pipérazin-1-ylméthyl)-cyclododécanol
1-(3-Méthoxy-benzyl)-2-(4-méthyl-pipérazin-1-ylméthyl)-cyclododécanol
1-(3-Fluoro-benzyl)-2-(4-méthyl-pipérazin-1-ylméthyl)-cyclododécanol
1-(2-Méthoxy-phényl)-2-(4-méthyl-pipérazin-1-ylméthyl)-cyclododécanol
1-(2-Méthyl-benzyl)-2-(4-méthyl-pipérazin-1-ylméthyl)-cyclododécanol
1-(3-Méthyl-benzyl)-2-(4-méthyl-pipérazin-1-ylméthyl)-cyclododécanol
1-(4-Chloro-benzyl)-2-(4-méthyl-pipérazin-1-ylméthyl)-cyclododécanol
1-(2,5-Diméthyl-benzyl)-2-(4-méthyl-pipérazin-1-ylméthyl)-cyclododécanol
1-(3-Chloro-benzyl)-2-(4-méthyl-pipérazin-1-ylméthyl)-cyclododécanol
1-(2,4-Dichloro-benzyl)-2-(4-méthyl-pipérazin-1-ylméthyl)-cyclododécanol
2-[4-(3-Chloro-phényl)-pipérazin-1-ylméthyl]-1-cyclohexylméthyl-cyclohexanol
2-[4-(3-Chloro-phényl)-pipérazin-1-ylméthyl]-1-(5-fluoro-2-méthoxy-phényl)-cyclohexanol
2-[4-(3-Chloro-phényl)-pipérazin-1-ylméthyl]-1-(3-fluoro-phényl)-cyclohexanol
1-(3-Chloro-phényl)-2-[4-(3-chloro-phényl)-pipérazin-1-ylméthyl]-cyclohexanol
2-[4-(3-Chloro-phényl)-pipérazin-1-ylméthyl]-1-(3,5-dichloro-phényl)-cyclohexanol
1-(2-Chloro-benzyl)-2-[4-(3-chloro-phényl)-pipérazin-1-ylméthyl]-cyclohexanol
2-[4-(3-Chloro-phényl)-pipérazin-1-ylméthyl]-1-(4-fluoro-benzyl)-cyclohexanol
2-[4-(3-Chloro-phényl)-pipérazin-1-ylméthyl]-1-(4-chloro-3-trifluorométhylphényl)-cyclohexanol
2-[4-(3-Chloro-phényl)-pipérazin-1-ylméthyl]-1-(3-fluoro-benzyl)-cyclohexanol
2-[4-(3-Chloro-phényl)-pipérazin-1-ylméthyl]-1-(2-méthoxy-phényl)-cyclohexanol
1-(3-Chloro-4-fluoro-phényl)-2-[4-(3-chloro-phényl)-pipérazin-1-ylméthyl]-cyclohexanol
2-[4-(3-Chloro-phényl)-pipérazin-1-ylméthyl]-1-(3-trifluorométhyl-phényl)-cyclohexanol
2-[4-(3-Chloro-phényl)-pipérazin-1-ylméthyi]-1-(3-méthyl-benzyl)-cyclohexanol
1-(4-Chloro-benzyl)-2-[4-(3-chloro-phényl)-pipérazin-1-ylméthyl]-cyclohexanol
1-(2-Chloro-6-fluoro-benzyl)-2-[4-(3-chloro-phényl)-pipérazin-1-ylméthyl]-cyclohexanol
2-[4-(3-Chloro-phényl)-pipérazin-1-ylméthyl]-1-(2,5-diméthyl-benzyl)-cyclohexanol
1-(3-Chloro-benzyl)-2-[4-(3-chloro-phényl)-pipérazin-1-ylméthyl]-cyclohexanol
2-[4-(2-Chloro-phényl)-pipérazin-1-ylméthyl]-1-phénylcyclohexanol
1-(4-Chloro-phényl)-2-[4-(2-chloro-phényl)-pipérazin-1-ylméthyl]-cyclohexanol
1-Benzyl-2-[4-(2-chloro-phényl)-pipérazin-1-ylméthyl]-cyclohexanol
2-[4-(2-Chloro-phényl)-pipérazin-1-ylméthyl]-1-(4-fluoro-3-méthyl-phényl)-cyclohexanol
2-[4-(2-Chloro-phényl)-pipérazin-1-ylméthyl]-1-o-tolyl-cyclohexanol
2-[4-(2-Chloro-phényl)-pipérazin-1-ylméthyl]-1-vinylcyclohexanol
1-(4-tert-Butyl-phényl)-2-[4-(2-chloro-phényl)-pipérazin-1-ylméthyl]-cyclohexanol
2-[4-(2-Chloro-phényl)-pipérazin-1-ylméthyl]-1-cyclopentyl-cyclohexanol
2-[4-(2-Chloro-phényl)-pipérazin-1-ylméthyl]-1-m-tolyl-cyclohexanol
2-[4-(2-Chloro-phényl)-pipérazin-1-ylméthyl]-bicyclohexyl-1-ol
2-[4-(2-Chloro-phényl)-pipérazin-1-ylméthyl]-1-(4-fluoro-phényl)-cyclohexanol
2-[4-(2-Chloro-phényl)-pipérazin-1-ylméthyl]-1-phénéthyl-cyclohexanol
2-[4-(2-Chloro-phényl)-pipérazin-1-ylméthyl]-1-phényléthynyl-cyclohexanol
2-[4-(2-Chloro-phényl)-pipérazin-1-ylméthyl]-1-thiophén-2-yl-cyclohexanol
2-[4-(2-Chloro-phényl)-pipérazin-1-ylméthyl]-1-(3-méthoxy-phényl)-cyclohexanol
2-[4-(2-Chloro-phényl)-pipérazin-1-ylméthyl]-1-(3-phényl-propyl)-cyclohexanol
2-[4-(2-Chloro-phényl)-pipérazin-1-ylméthyl]-1-p-tolyl-cyclohexanol
2-[4-(2-Chloro-phényl)-pipérazin-1-ylméthyl]-1-(4-méthoxy-phényl)-cyclohexanol
2-[4-(2-Chloro-phényl)-pipérazin-1-ylméthyl]-1-cyclohexylméthyl-cyclohexanol
2-[4-(2-Chloro-phényl)-pipérazin-1-ylméthyl]-1-(5-fluoro-2-méthoxy-phényl)-cyclohexanol
1-(3-Chloro-phényl)-2-[4-(2-chloro-phényl)-pipérazin-1-ylméthyl]-cyclohexanol
2-[4-(2-Chloro-phényl)-pipérazin-1-ylméthyl]-1-(3,5-dichloro-phényl)-cyclohexanol
1-(2-Chloro-benzyl)-2-[4-(2-chloro-phényl)-pipérazin-1-ylméthyl]-cyclohexanol
2-[4-(2-Chloro-phényl)-pipérazin-1-ylméthyl]-1-(4-fluoro-benzyl)-cyclohexanol
2-[4-(2-Chloro-phényl)-pipérazin-1-ylméthyl]-1-(3-méthoxy-benzyl)-cyclohexanol
2-[4-(2-Chloro-phényl)-pipérazin-1-ytméthyl]-1-(4-chloro-3-trifluorométhyl-phényl)-cyclohexanol
2-[4-(2-Chloro-phényl)-pipérazin-1-ylméthyl]-1-(3-fluoro-benzyl)-cyclohexanol
2-[4-(2-Chloro-phényl)-pipérazin-1-ylméthyl]-1-(2-méthoxy-phényl)-cyclohexanol
2-[4-(2-Chloro-phényl)-pipérazin-1-ylméthyl]-1-(2-méthyl-benzyl)-cyclohexanol
1-(3-Chloro-4-fluoro-phényl)-2-[4-(2-chloro-phényl)-pipérazin-1-ylméthyl]-cyclohexanol
2-[4-(2-Chloro-phényl)-pipérazin-1-ylméthyl]-1-(3-trifluorométhyl-phényl)-cyclohexanol
2-[4-(2-Chloro-phényl)-pipérazin-1-ylméthyl]-1-(3-méthyl-benzyl)-cyclohexanol
1-(4-Chloro-benzyl)-2-[4-(2-chloro-phényl)-pipérazin-1-ylméthyl]-cyclohexanol
1-(2-Chloro-6-fluoro-benzyl)-2-[4-(2-chloro-phényl)-pipérazin-1-ylméthyl]-cyclohexanol
2-[4-(2-Chloro-phényl)-pipérazin-1-ylméthyl]-1-(2,5-diméthyl-benzyl)-cyclohexanol
1-(3-Chloro-benzyl)-2-[4-(2-chloro-phényl)-pipérazin-1-ylméthyl]-cyclohexanol
2-[4-(2-Chloro-phényl)-pipérazin-1-ylméthyl]-1-(2,4-dichloro-benzyl)-cyclohexanol
2-[4-(2-méthoxy-phényl)-pipérazin-1-ylméthyl]-1-phényl-cyclohexanol
1-(4-chloro-phényl)-2-[4-(2-méthoxy-phényl)-pipérazin-1-ylméthyl]-cyclohexanol
1-Benzyl-2-[4-(2-méthoxy-phényl)-pipérazin-1-ylméthyl]-cyclohexanol
1-(4-Fluoro-3-méthyl-phényl)-2-[4-(2-méthoxy-phényl)-pipérazin-1-ylméthyl]-cyclohexanol
2-[4-(2-Méthoxy-phényl)-pipérazin-1-ylméthyl]-1-o-tolyl-cyclohexanol
2-[4-(2-Méthoxy-phényl)-pipérazin-1-ylméthyl]-1-vinylcyclohexanol
1-(4-tert-Butyl-phényl)-2-[4-(2-méthoxy-phényl)-pipérazin-1-ylméthyl]-cyclohexanol
1-Cyclopentyl-2-[4-(2-méthoxy-phényl)-pipérazin-1-ylméthyl]-cyclohexanol
2-[4-(2-Méthoxy-phényl)-pipérazin-1-ylméthyl]-1-m-tolyl-cyclohexanol
2-[4-(2-Méthoxy-phényl)-pipérazin-1-ylméthyl]-bicyclohexyl-1-ol
1-(4-Fluoro-phényl)-2-[4-(2-méthoxy-phényl)-pipérazin-1-ylméthyl]-cyclohexanol
2-[4-(2-Méthoxy-phényl)-pipérazin-1-ylméthyl]-1-phénéthyl-cyclohexanol
2-[4-(2-Méthoxy-phényl)-pipérazin-1-ylméthyl]-1-phényléthynyl-cyclohexanol
2-[4-(2-Méthoxy-phényl)-pipérazin-1-ylméthyl]-1-thiophén-2-yl-cyclohexanol
1-(3-Méthoxy-phényl)-2-[4-(2-méthoxy-phényl)-pipérazin-1-ylméthyl]-cyclohexanol
2-[4-(2-Méthoxy-phényl)-pipérazin-1-ylméthyl]-1-(3-phényl-propyl)-cyclohexanol
1-(2,3-Dichloro-phényl)-2-[4-(2-méthoxy-phényl)-pipérazin-1-ylméthyl]-cyclohexanol
2-[4-(2-Méthoxy-phényl)-pipérazin-1-ylméthyl]-1-p-tolyl-cyclohexanol
1-(4-Méthoxy-phényl)-2-[4-(2-méthoxy-phényl)-pipérazin-1-ylméthyl]-cyclohexanol
1-Cyclohexylméthyl-2-[4-(2-méthoxy-phényl) -pipérazin-1-ylméthyl]-cyclohexanol
1-(5-Fluoro-2-méthoxy-phényl)-2-[4-(2-méthoxy-phényl)-pipérazin-1-ylméthyl]-cyclohexanol
1-(3-Fluoro-phényl)-2-[4-(2-méthoxy-phényl)-pipérazin-1-ylméthyl]-cyclohexanol
1-(3-Chloro-phényl)-2-[4-(2-méthoxy-phényl)-pipérazin-1-ylméthyl]-cyclohexanol
1-(3,5-Dichloro-phényl)-2-[4-(2-méthoxy-phényl)-pipérazin-1-ylméthyl]-cyclohexanol
1-(2-Chloro-benzyl)-2-[4-(2-méthoxy-phényl)-pipérazin-1-ylméthyl]-cyclohexanol
1-(4-Fluoro-benzyl)-2-[4-(2-méthoxy-phényl)-pipérazin-1-ylméthyl]-cyclohexanol
1-(3-Méthoxy-benzyl)-2-[4-(2-méthoxy-phényl)-pipérazin-1-ylméthyl]-cyclohexanol
1-(4-Chloro-3-trifluorométhyl-phényl)-2-[4-(2-méthoxyphényl)-pipérazin-1-ylméthyl]-cyclohexanol
1-(3-Fluoro-benzyl)-2-[4-(2-méthoxy-phényl)-pipérazin-1-ylméthyl]-cyclohexanol
1-(2-Méthoxy-phényl)-2-[4-(2-méthoxy-phényl)-pipérazin-1-ylméthyl]-cyclohexanol
2-[4-(2-Méthoxy-phényl)-pipérazin-1-ylméthyl]-1-(2-méthyl-benzyl)-cyclohexanol
1-(3-Chloro-4-fluoro-phényl)-2-[4-(2-méthoxy-phényl)-pipérazin-1-ylméthyl]-cyclohexanol
2-[4-(2-Méthoxy-phényl)-pipérazin-1-ylméthyl]-1-(3-trifluorométhyl-phényl)-cyclohexanol
2-[4-(2-Méthoxy-phényl)-pipérazin-1-ylméthyl]-1-(3-méthyl-benzyl)-cyclohexanol
1-(4-Chloro-benzyl)-2-[4-(2-méthoxy-phényl)-pipérazin-1-ylméthyl]-cyclohexanol
1-(2-Chloro-6-fluoro-benzyl)-2-[4-(2-méthoxy-phényl)-pipérazin-1-ylméthyl]-cyclohexanol
1-(2,5-Diméthyl-benzyl)-2-[4-(2-méthoxy-phényl)-pipérazin-1-ylméthyl]-cyclohexanol
1-(3-Chloro-benzyl)-2-[4-(2-méthoxy-phényl)-pipérazin-1-ylméthyl]-cyclohexanol
1-(2,4-Dichloro-benzyl)-2-[4-(2-méthoxy-phényl)-pipérazin-1-ylméthyl]-cyclohexanol
1-Benzyl-2-[4-(2-méthoxy-phényl)-pipérazin-1-ylméthyl]-cyclododécanol
2-[4-(2-Méthoxy-phényl)-pipérazin-1-ylméthyl]-1-vinylcyclododécanol
1-Phénéthyl-2-[phényl-(4-phényl-pipérazin-1-yl)-méthyl]-cyclohexanol
1-Benzyl-2-[(4-méthyl-pipérazin-1-yl)-phényl-méthyl]-cyclohexanol
1-Benzyl-2-[phényl-(4-phényl-pipérazin-1-yl)-méthyl]-cyclohexanol
2-[(4-Méthyl-pipérazin-1-yl)-phényl-méthyl]-1-phénéthyl-cyclohexanol et
2-[4-(2-Méthoxy-phényl)-pipérazin-1-ylméthyl]-1-phénéthyl-cyclododécanol
sous forme de leurs diastéréoisomères, de leurs énantiomères et de leurs mélanges, y compris leurs racémates, ainsi que sous forme de bases, sels et solvates correspondants.
